# EUROPÄISCHE PATENTSCHRIFT

(11) **EP 2 794 619 B1**
(45) Veröffentlichungstag und Bekanntmachung des Hinweises auf die Patenterteilung: **14.03.2018**
(21) Anmeldenummer: 12791107.1
(22) Anmeldetag: 26.11.2012
(51) Int. Cl.: C07F 5/02, H01L 51/00

(54) **VERBINDUNGEN FÜR ORGANISCHE ELEKTROLUMINESZENZVORRICHTUNGEN**
COMPOUNDS FOR ORGANIC ELECTROLUMINESCENT DEVICES
COMPOSÉS DESTINÉS À DES DISPOSITIFS ÉLECTROLUMINESCENTS ORGANIQUES

(30) Priorität: 22.12.2011 EP 11010103
(43) Veröffentlichungstag der Anmeldung: 29.10.2014
(73) Patentinhaber: Merck Patent GmbH, 64293 Darmstadt (DE)
(72) Erfinder: MUJICA-FERNAUD, Teresa, 64283 Darmstadt (DE); PARHAM, Amir Hossain, 65929 Frankfurt am Main (DE); STOESSEL, Philipp, 60487 Frankfurt am Main (DE); PFLUMM, Christof, 64291 Darmstadt (DE); BUESING, Arne, 65929 Frankfurt am Main (DE); EBERLE, Thomas, 76829 Landau (DE)
(86) Internationale Anmeldenummer: PCT/EP2012/004879
(87) Internationale Veröffentlichungsnummer: WO 2013/091762

(56) Entgegenhaltungen:
- WO-A1-2010/108579
- LOTHAR WEBER ET AL: "Synthetic, Structural, Photophysical and Computational Studies on [pi]-Conjugated 1,3,2-Benzodiazaboroles with Carbazole Building Blocks", EUROPEAN JOURNAL OF INORGANIC CHEMISTRY, Bd. 2010, Nr. 34, 1. Dezember 2010 (2010-12-01), Seiten 5416-5425, XP055050349, ISSN: 1434-1948, DOI: 10.1002/ejic.201000665
- LOTHAR WEBER ET AL: "Synthetic, structural, photophysical and computational studies of [pi]-conjugated bis- and tris-1,3,2-benzodiazaboroles and related bis(boryl) dithiophenes", DALTON TRANSACTIONS, Nr. 8, 1. Januar 2009 (2009-01-01), Seite 1339, XP055050389, ISSN: 1477-9226, DOI: 10.1039/b815931a

## Beschreibung

Die vorliegende Erfindung betrifft Materialien für die Verwendung in elektronischen Vorrichtungen, Verfahren zur Herstellung dieser Materialien sowie elektronische Vorrichtunen, insbesondere organische Elektrolumineszenzvorrichtungen, enthaltend diese Materialien.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission zeigen, jedoch immer noch Verbesserungsbedarf, insbesondere im Hinblick auf Effizienz, Betriebsspannung und Lebensdauer. Dies gilt insbesondere für OLEDs, welche im kürzerwelligen Bereich, also grün oder blau, emittieren.

Die Eigenschaften phosphoreszierender OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden unter anderem Ketone (z. B. gemäß WO 2004/093207 oder WO 2010/006680) als Matrixmaterialien für phosphoreszierende Emitter verwendet. Weiterhin sind als Matrixmaterialien für phosphoreszierende Emitter Boronsäurederivate (z. B. gemäß WO 2006/117052. WO 2010/108579) oder Diazaphospholderivate (z. B. gemäß WO 2010/054730) bekannt.

Weiterhin sind Benzodiazaborolderivate umfassend Carbazol- oder Thiophen-Gruppen als Emitter oder Elektronentransportmaterialien bekannt z. B. gemäß WEBER, L. et al. Synthetic, structural, photophysical and computational studies on π-conjugated 1,3,2-benzodiazaboroles with carbazole building blocks, Eur. J. Inor. Chem. 2010, pages 5416-5425 und gemäß WEBER, L. et al. Synthetic, structural, photophysical and computational studies of π-conjugated bis- and tris-1,3,2-benzodiazaboroles and related bis(boryl) dithiophenes, Dalton Trans. 2009, pages 1339-1351).

Allerdings besteht bei Verwendung all dieser Matrixmaterialien ebenso wie bei anderen Matrixmaterialien noch Verbesserungsbedarf, insbesondere in Bezug auf die Effizienz und die Lebensdauer der Vorrichtung.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Lochtransport-/ Elektronenblockiermaterial bzw. Exzitonenblockiermaterial oder als Elektronentransport- bzw. Lochblockiermaterial. Insbesondere ist es die Aufgabe der vorliegenden Erfindung, Matrixmaterialien bereitzustellen, welche sich potenziell auch für blau und grün phosphoreszierende OLEDs eignen.

Überraschend wurde gefunden, dass die unten näher beschriebenen Verbindungen diese Aufgabe lösen und zu Verbesserungen der organischen Elektrolumineszenzvorrichtung führen, insbesondere hinsichtlich der Lebensdauer, der Effizienz und/oder der Betriebsspannung. Dies gilt insbesondere bei Einsatz der erfindungsgemäßen Verbindungen als Matrixmaterial. Dabei wurde insbesondere gefunden, dass die erfindungsgemäßen Verbindungen bessere Ergebnisse ergeben als die in WO 2006/117052 beschriebenen Boronsäurederivate. Diese Verbindungen sowie organische Elektrolumineszenzvorrichtungen, welche derartige Verbindungen enthalten, sind daher der Gegenstand der vorliegenden Erfindung.

Gegenstand der vorliegenden Erfindung ist eine Verbindung gemäß der folgenden Formel (1), 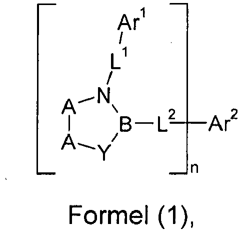 wobei für die verwendeten Symbole und Indizes gilt:
- A-A: ist gleich oder verschieden bei jedem Auftreten eine Einheit der folgenden Formel (2), (3), (4), (5), (6), (7) oder (8), 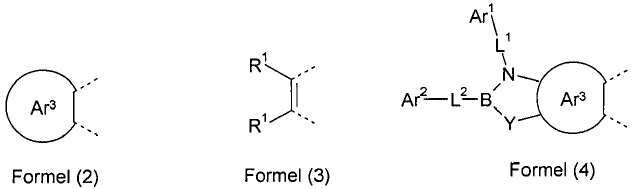 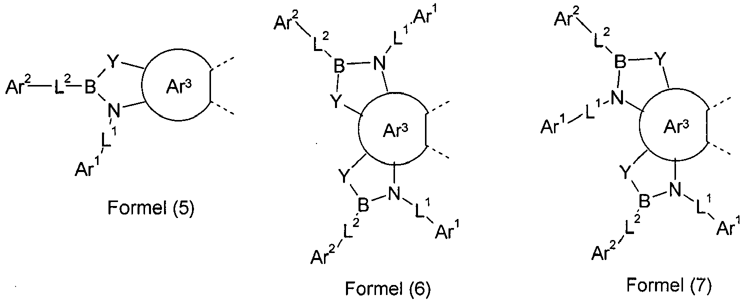 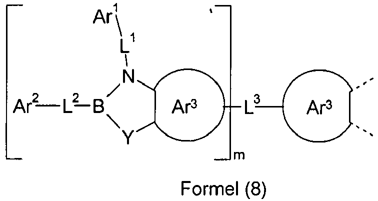 wobei die gestrichelte Bindung jeweils die Verknüpfung mit N bzw. Y darstellt;
- Y: ist gleich oder verschieden bei jedem Auftreten N-R², O oder S;
- Ar¹, Ar²: ist bei jedem Auftreten gleich oder verschieden eine Aryl- oder Heteroarylgruppe mit 5 bis 18 aromatischen Ringatomen, welche durch einen oder mehrere Reste R¹ substituiert sein kann; mit der Maßgabe, dass die Heteroatome aus N. O und/oder S ausgewählt sind, wenn Ar¹ oder Ar² eine Heteroarylgruppe ist:
- Ar³: ist bei jedem Auftreten gleich oder verschieden eine Aryl- oder Heteroarylgruppe mit 5 bis 14 aromatischen Ringatomen, welche durch einen oder mehrere Reste R¹ substituiert sein kann;
- L¹, L²: ist bei jedem Auftreten gleich oder verschieden eine Einfachbindung oder ein bivalentes aromatisches oder heteroaromatisches Ringsystem mit 5 bis 24 aromatischen Ringatomen, das durch einen oder mehrere Reste R¹ substituiert sein kann;
- L³: ist eine Einfachbindung oder eine bivalente, trivalente oder tetravalente Gruppe;
- R¹: ist bei jedem Auftreten gleich oder verschieden ausgewählt aus der Gruppe bestehend aus H, D, F, Cl, Br, I, CN, NO₂, N(R³)₂, C(=O)R³, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C≡C, Si(R³)₂, Ge(R³)₂, Sn(R³)₂, C=O, C=S, C=Sₑ, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
- R²: ist bei jedem Auftreten gleich oder verschieden ausgewählt aus der Gruppe bestehend aus einer geradkettigen Alkylgruppe mit 1 bis 40 C-Atomen oder einer verzweigten oder cyclischen Alkylgruppe mit 3 bis 40 C-Atomen, die jeweils mit einem oder mehreren Resten R³ substituiert sein kann, wobei eine oder mehrere nicht-benachbarte CH₂-Gruppen durch R³C=CR³, C≡C oder C=O ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, oder einer Kombination dieser Systeme; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein;
- R³: ist ausgewählt aus der Gruppe bestehend aus H, D, F, CN, aliphatischem Kohlenwasserstoffrest mit 1 bis 20 C-Atomen, aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches, aromatisches oder heteroaromatisches Ringsystem bilden können;
- n: ist 1, 2, 3, 4, 5 oder 6;
- m: ist 1, wenn L³ eine Einfachbindung oder eine bivalente Gruppe ist, oder ist 2, wenn L³ eine trivalente Gruppe ist, oder ist 3, wenn L³ eine tetravalente Gruppe ist;
dadurch gekennzeichnet, dass mindestens eine Gruppe Ar¹ vorhanden ist, die für eine Heteroarylgruppe mit 5 bis 18 aromatischen Ringatomen steht, welche auch durch einen oder mehrere Reste R¹ substituiert sein kann, oder dass mindestens eine Gruppe Ar² vorhanden ist, die für eine elektronenarme Heteroarylgruppe mit 5 bis 18 aromatischen Ringatomen steht, wobei eine elektronenarme Heteroarylgruppe entweder eine 5-Ring-Heteroarylgruppe mit mindestens zwei Heteroatomen oder eine 6-Ring-Heteroarylgruppe mit mindestens einem Heteroatom ist, wobei an diese Gruppen weitere 6-Ring-Aryl- oder 6-Ring-Heteroarylgruppen ankondensiert sein können.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc.. Dabei können an diese Gruppen auch noch weitere 6-Ring-Aryl- oder 6-Ring-Heteroarylgruppen ankondensiert sein, wie dies beispielsweise in Benzimidazol, Chinolin oder Phenanthrolin der Fall ist.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, N-oder O-Atom, unterbrochen sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe unterbrochen sind.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch R³C=CR³, C≡C, Si(R³)₂, Ge(R³)₂, Sn(R³)₂, C=O, C=S, C=Se, C=NR³, P(=O)(R³), SO, SO₂, NR³, O, S oder CONR³ ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R³ oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis-oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Wenn die Verbindung der Formel (1) eine Einheit gemäß einer oder mehrerer der Formeln (2) oder (4) bis (8) enthält, so steht Ar³ bevorzugt gleich oder verschieden bei jedem Auftreten für eine Aryl- oder Heteroarylgruppe mit 5 bis 10 aromatischen Ringatomen, besonders bevorzugt mit 5 oder 6 aromatischen Ringatomen. Bevorzugte Aryl- und Heteroarylgruppen Ar³ sind gleich oder verschieden bei jedem Auftreten ausgewählt aus der Gruppe bestehend aus Benzol, Pyridin, Pyrimidin, Pyridazin, Pyrazin, Furan, Thiophen, Pyrrol, Naphthalin, Phenanthren, Chinolin, Isochinolin, Chinoxalin, Indol, Benzofuran und Benzothiophen. Dabei enthält die Aryl- oder Heteroarylgruppe bevorzugt keine direkt aneinander kondensierten Sechsringe. Besonders bevorzugte Aryl- und Heteroarylgruppen Ar³ sind gleich oder verschieden bei jedem Auftreten ausgewählt aus der Gruppe bestehend aus Benzol, Pyridin, Pyrimidin und Pyrazin, insbesondere Benzol.

Bevorzugte Ausführungsformen der Verbindungen gemäß der oben aufgeführten Formel (1) sind die Verbindungen der Formeln (9) bis (18), 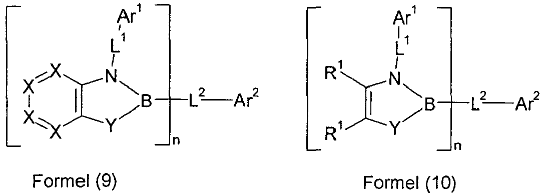 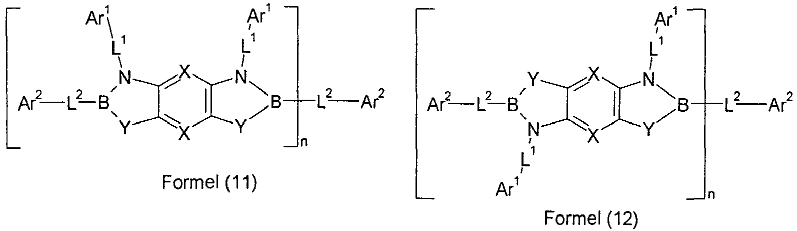 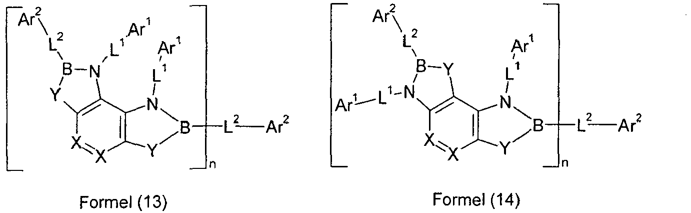 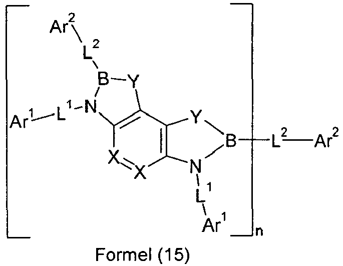 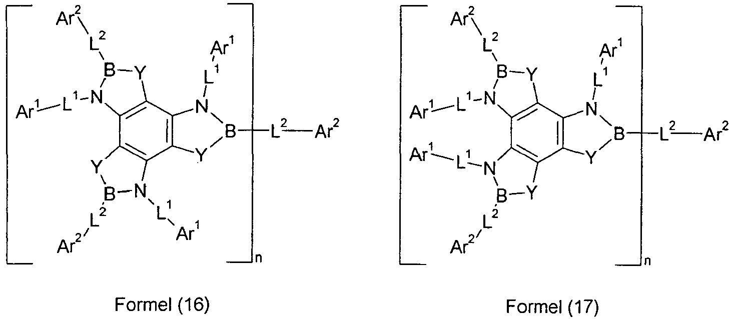 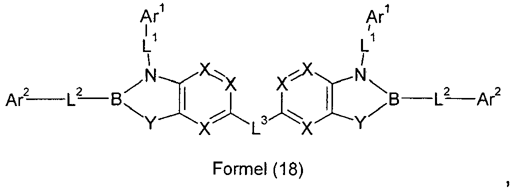 wobei X bei jedem Auftreten gleich oder verschieden CR¹ oder N ist und die weiteren die verwendeten Symbole und Indizes die oben genannten Bedeutungen aufweisen.

In einer bevorzugten Ausführungsform der Verbindungen gemäß Formel (1) bzw. Formel (9) bis (18) steht das Symbol Y gleich oder verschieden bei jedem Auftreten für N-R². Entsprechend steht auch bevorzugt das Symbol Y in den Untereinheiten gemäß den Formeln (4) bis (8) gleich oder verschieden bei jedem Auftreten für N-R².

In einer weiteren bevorzugten Ausführungsform der Erfindung steht L¹ bzw. L² gleich oder verschieden bei jedem Auftreten für eine Einfachbindung oder eine bivalente Arylen- oder Heteroarylengruppe mit 5 bis 10 aromatischen Ringatomen, die jeweils durch einen oder mehrere Reste R¹ substituiert sein kann. Besonders bevorzugt steht L¹ bzw. L² gleich oder verschieden bei jedem Auftreten für eine bivalente Arylen- oder Heteroarylengruppe mit 6 aromatischen Ringatomen, die durch einen oder mehrere Reste R¹ substituiert sein kann, bevorzugt aber unsubstituiert ist, insbesondere für 1,2-, 1,3- oder 1,4-Phenylen, Pyridin, Pyrimidin oder Triazin, ganz besonders bevorzugt für Phenylen oder Pyridin.

In einer weiteren bevorzugten Ausführungsform der Verbindungen gemäß Formel (1) bzw. Formel (18) steht L³ für eine Einfachbindung, O, S, NR², eine Alkylengruppe mit 1 bis 10 C-Atomen, welche mit einem oder mehreren Resten R³ substituiert sein kann, oder ein aromatisches oder heteroaromatisches Ringsystem mit 5 bis 30 aromatischen Ringatomen, welches mit einem oder mehreren Resten R³ substituiert sein kann.

In einer weiteren bevorzugten Ausführungsform der Erfindung stehen maximal zwei Symbole X in jedem Cyclus für N und die anderen Symbole X stehen gleich oder verschieden bei jedem Auftreten für CR¹. Besonders bevorzugt steht maximal ein Symbol X in jedem Cyclus für N und die anderen Symbole X stehen gleich oder verschieden bei jedem Auftreten für CR¹. Ganz besonders bevorzugt stehen alle Symbole X gleich oder verschieden bei jedem Auftreten für CR¹.

In einer weiteren bevorzugten Ausführungsform der Erfindung steht die Gruppe Ar¹ bzw. Ar² gleich oder verschieden bei jedem Auftreten für ein aromatisches oder heteroaromatisches Ringsystem mit 5 bis 24 aromatischen Ringatomen, besonders bevorzugt mit 5 bis 18 aromatischen Ringatomen, wobei das aromatische oder heteroaromatische Ringsystem jeweils auch durch einen oder mehrere Reste R¹ substituiert sein kann. Besonders bevorzugt handelt es sich bei Ar¹ bzw. Ar² gleich oder verschieden bei jedem Auftreten um eine Aryl- oder Heteroarylgruppe mit 5 bis 13 aromatischen Ringatomen, die jeweils auch durch einen oder mehrere Reste R¹ substituiert sein kann. Dabei enthält Ar¹ bzw. Ar² bevorzugt keine Aryl- oder Heteroarylgruppen mit mehr als zwei direkt aneinander kondensierten Sechsringen und enthält besonders bevorzugt überhaupt keine direkt aneinander kondensierte Sechsringe. Bevorzugte Gruppen Ar¹ bzw. Ar² sind daher aufgebaut aus jeweils einer oder mehreren der Gruppen Benzol, Pyridin, Pyrimidin, Pyridazin, Pyrazin, Triazin, Pyrrol, Thiophen, Furan, Imidazol, Indol, Benzothiophen, Benzofuran, Benzimidazol, Carbazol, Dibenzofuran oder Dibenzothiophen. Besonders bevorzugte Gruppen Ar sind aufgebaut aus jeweils einer oder mehreren Gruppen Benzol, Pyridin, Pyrimidin, Pyridazin, Pyrazin, Triazin oder Benzimidazol.

Wie oben beschrieben, sind die erfindungsgemäßen Verbindungen der Formel (1) dadurch charakterisiert, dass mindestens eine Gruppe Ar¹ eine Heteroarylgruppe darstellt oder dass mindestens eine Gruppe Ar² eine elektronenarme Heteroarylgruppe darstellt. In einer bevorzugten Ausführungsform handelt es sich auch bei Ar¹, wenn diese Gruppe für eine Heteroarylgruppe steht, um eine elektronenarme Heteroarylgruppe.

Bevorzugte elektronenarme Heteroarylgruppen Ar¹ bzw. Ar² sind ausgewählt aus der Gruppe bestehend aus Pyridin, Pyrimidin, Pyrazin, Pyridazin, Imidazol, Triazol, Oxadiazol oder Benzimidazol, welche über eine beliebige Position an L¹ bzw. L² gebunden ist und welche durch einen oder mehrere Reste R¹ substituiert sein kann. Besonders bevorzugt elektronenarme Heteroarylgruppen Ar¹ bzw. Ar² sind ausgewählt aus der Gruppe bestehend aus Pyridin, Pyrimidin, Triazin oder Benzimidazol, welches jeweils über eine beliebige Position an L¹ bzw. L² gebunden ist und welche durch einen oder mehrere Reste R¹ substituiert sein kann.

In einer bevorzugten Ausführungsform der Erfindung ist die Einheit A-A ausgewählt aus den Strukturen gemäß Formel (2) oder (3), insbesondere gemäß Formel (2).

In einer weiteren bevorzugten Ausführungsform der Erfindung ist der Index n = 1, 2, 3 oder 4, besonders bevorzugt 1, 2 oder 3, ganz besonders bevorzugt 1 oder 2.

In einer weiteren bevorzugten Ausführungsform der Erfindung ist der Rest R¹ gleich oder verschieden bei jedem Auftreten ausgewählt aus der Gruppe bestehend aus H, D, F, CN, N(R³)₂, einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen oder einer Alkenylgruppe mit 2 bis 20 C-Atomen, die jeweils mit einem oder mehreren Resten R³ substituiert sein kann, wobei eine oder mehrere nicht-benachbarte CH₂-Gruppen durch R³C=CR³ oder O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R³ substituiert sein kann, einer Aryloxy- oder Heteroaryloxygruppe mit 5 bis 30 aromatischen Ringatomen, die mit einem oder mehreren Resten R³ substituiert sein können, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann. Besonders bevorzugt ist der Rest R¹ gleich oder verschieden bei jedem Auftreten ausgewählt aus der Gruppe bestehend aus H, D, CN, F, einer geradkettigen Alkylgruppe mit 1 bis 10 C-Atomen, besonders bevorzugt mit 1 bis 4 C-Atomen, oder einer verzweigten oder cyclischen Alkylgruppe mit 3 bis 10 C-Atomen, besonders bevorzugt mit 3 bis 6 C-Atomen, oder einer Alkenylgruppe mit 2 bis 10 C-Atomen, besonders bevorzugt mit 2 bis 4 C-Atomen, die jeweils mit einem oder mehreren Resten R³ substituiert sein kann, wobei ein oder mehrere H-Atome durch D ersetzt sein können, einem aromatischen oder heteroaromatischen Ringsystem mit 5 bis 12 aromatischen Ringatomen, das jeweils mit einem oder mehreren Resten R³ substituiert sein kann, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann.

In einer weiteren bevorzugten Ausführungsform der Erfindung ist der Rest R² bei jedem Auftreten gleich oder verschieden ausgewählt aus der Gruppe bestehend aus einem aromatischen oder heteroaromatischen Ringsystem mit 5 bis 30 aromatischen Ringatomen, das jeweils mit einem oder mehreren Resten R³ substituiert sein kann; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein kann. In einer besonders bevorzugten Ausführungsform der Erfindung steht R² für Phenyl, Naphthyl, Biphenyl, Terphenyl oder Quaterphenyl, welches jeweils mit einem oder mehreren Resten R³ substituiert sein kann, insbesondere für Phenyl oder Biphenyl, welches jeweils mit einem oder mehreren Resten R³ substituiert sein kann, jedoch bevorzugt unsubstituiert ist.

Besonders bevorzugt sind Verbindungen der oben aufgeführten Formeln (1) bzw. (8) bis (18), in denen die oben genannten Bevorzugungen gleichzeitig gelten. Besonders bevorzugt sind daher Verbindungen, in denen R³ wie vorne definiert ist und weiterhin gilt:
- Y: ist gleich oder verschieden bei jedem Auftreten für N-R²;
- L¹, L²: ist gleich oder verschieden bei jedem Auftreten eine Einfachbindung oder eine bivalente Arylen- oder Heteroarylengruppe mit 5 bis 10 aromatischen Ringatomen, die jeweils durch einen oder mehrere Reste R¹ substituiert sein kann.
- L³: ist eine Einfachbindung, O, S, NR², eine Alkylengruppe mit 1 bis 10 C-Atomen, welche mit einem oder mehreren Resten R³ substituiert sein kann, oder ein aromatisches oder heteroaromatisches Ringsystem mit 5 bis 30 aromatischen Ringatomen, welches mit einem oder mehreren Resten R³ substituiert sein kann;
- X: ist bei jedem Auftreten gleich oder verschieden CR¹ oder N, wobei in jedem Cyclus maximal zwei Symbole X für N stehen und die anderen Symbole X gleich oder verschieden bei jedem Auftreten für CR¹ stehen;
- Ar¹, Ar²: steht gleich oder verschieden bei jedem Auftreten für ein aromatisches oder heteroaromatisches Ringsystem mit 5 bis 24 aromatischen Ringatomen, besonders bevorzugt mit 5 bis 18 aromatischen Ringatomen, wobei das aromatische oder heteroaromatische Ringsystem jeweils auch durch einen oder mehrere Reste R¹ substituiert sein kann; dabei ist mindestens eine Gruppe Ar¹ eine Heteroarylgruppe bzw. mindestens eine Gruppe Ar² eine elektronenarme Heteroarylgruppe, die durch einen oder mehrere Reste R¹ substituiert sein kann; mit der Maßgabe, dass die Heteroatome aus N, O und/oder S ausgewählt sind, wenn Ar¹ oder Ar² eine heteroarylgruppe ist;
- n: ist 1, 2, 3 oder 4, bevorzugt 1, 2 oder 3;
- R¹: ist gleich oder verschieden bei jedem Auftreten ausgewählt aus der Gruppe bestehend aus H, D, F, CN, N(R³)₂, einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen oder einer Alkenylgruppe mit 2 bis 20 C-Atomen, die jeweils mit einem oder mehreren Resten R³ substituiert sein kann, wobei eine oder mehrere nicht-benachbarte CH₂-Gruppen durch R³C=CR³ oder O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R³ substituiert sein kann, einer Aryloxy- oder Heteroaryloxygruppe mit 5 bis 30 aromatischen Ringatomen, die mit einem oder mehreren Resten R³ substituiert sein können, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
- R²: ist bei jedem Auftreten gleich oder verschieden ausgewählt aus der Gruppe bestehend aus einem aromatischen oder heteroaromatischen Ringsystem mit 5 bis 30 aromatischen Ringatomen, das jeweils mit einem oder mehreren Resten R³ substituiert sein kann; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein kann.

Ganz besonders bevorzugt sind Verbindungen gemäß Formel (1) bzw. Formel (8) bis (18), in denen R³ wie vorne definiert ist und weiterhin gilt:
- Y: ist gleich oder verschieden bei jedem Auftreten für N-R²;
- L¹, L²: ist gleich oder verschieden bei jedem Auftreten eine bivalente Arylen- oder Heteroarylengruppe mit 6 aromatischen Ringatomen, die durch einen oder mehrere Reste R¹ substituiert sein kann, bevorzugt aber unsubstituiert ist, insbesondere für 1,2-, 1,3- oder 1,4-Phenylen, Pyridin, Pyrimidin oder Triazin;
- L³: ist eine Einfachbindung, O, S, NR², eine Alkylengruppe mit 1 bis 10 C-Atomen, welche mit einem oder mehreren Resten R³ substituiert sein kann, oder ein aromatisches oder heteroaromatisches Ringsystem mit 5 bis 30 aromatischen Ringatomen, welches mit einem oder mehreren Resten R³ substituiert sein kann;
- X: ist bei jedem Auftreten gleich oder verschieden CR¹ oder N, wobei in jedem Cyclus maximal ein Symbol X und bevorzugt überhaupt kein Symbol X für N stehen und die anderen Symbole X gleich oder verschieden bei jedem Auftreten für CR¹ stehen;
- Ar¹, Ar²: steht gleich oder verschieden bei jedem Auftreten für eine Aryl- oder Heteroarylgruppe mit 5 bis 13 aromatischen Ringatomen, die jeweils auch durch einen oder mehrere Reste R¹ substituiert sein kann und die keine Aryl- oder Heteroarylgruppen mit mehr als zwei direkt aneinander kondensierten Sechsringen enthält, insbesondere ausgewählt aus der Gruppe bestehend aus jeweils einer oder mehreren der Gruppen Benzol, Pyridin, Pyrimidin, Pyridazin, Pyrazin, Triazin, Pyrrol, Thiophen, Furan, Imidazol, Indol, Benzothiophen, Benzofuran, Benzimidazol, Carbazol, Dibenzofuran oder Dibenzothiophen; dabei ist mindestens eine Gruppe Ar¹ oder Ar² eine elektronenarme Heteroarylgruppe, die durch einen oder mehrere Reste R¹ substituiert sein kann, die ausgewählt ist aus der Gruppe bestehend aus Pyridin, Pyrimidin, Pyrazin, Pyridazin, Imidazol, Triazol, Oxadiazol oder Benzimidazol, welche über eine beliebige Position an L¹ bzw. L² gebunden ist und welche durch einen oder mehrere Reste R¹ substituiert sein kann;
- n: ist 1 oder 2;
- R¹: ist gleich oder verschieden bei jedem Auftreten ausgewählt aus der Gruppe bestehend aus H, D, CN, F, einer geradkettigen Alkylgruppe mit 1 bis 10 C-Atomen, besonders bevorzugt mit 1 bis 4 C-Atomen, oder einer verzweigten oder cyclischen Alkylgruppe mit 3 bis 10 C-Atomen, besonders bevorzugt mit 3 bis 6 C-Atomen, oder einer Alkenylgruppe mit 2 bis 10 C-Atomen, besonders bevorzugt mit 2 bis 4 C-Atomen, die jeweils mit einem oder mehreren Resten R³ substituiert sein kann, wobei ein oder mehrere H-Atome durch D ersetzt sein können, einem aromatischen oder heteroaromatischen Ringsystem mit 5 bis 12 aromatischen Ringatomen, das jeweils mit einem oder mehreren Resten R³ substituiert sein kann, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
- R²: ist bei jedem Auftreten gleich oder verschieden ausgewählt aus der Gruppe bestehend aus Phenyl, Naphthyl, Biphenyl, Terphenyl oder Quaterphenyl, welches jeweils mit einem oder mehreren Resten R³ substituiert sein kann, insbesondere für Phenyl oder Biphenyl, welches jeweils mit einem oder mehreren Resten R³ substituiert sein kann, jedoch bevorzugt unsubstituiert ist.

Für Verbindungen, die aus Lösung verarbeitet werden, eignen sich als Substituenten insbesondere auch lange Alkylgruppen, beispielsweise mit 5 bis 10 C-Atomen, oder substituierte oder unsubstituierte Oligoarylengruppen. Geeignete Oligoarylengruppen sind zum Beispiel Terphenyl, insbesondere meta-Terphenyl oder verzweigtes Terphenyl, meta-Quaterphenyl oder verzweigtes Quaterphenyl.

Beispiele für bevorzugte Verbindungen gemäß den oben aufgeführten Ausführungsformen bzw. Verbindungen, wie sie bevorzugt in organischen elektronischen Vorrichtungen eingesetzt werden können, sind die Verbindungen der folgenden Strukturen (1) bis (50).

| | |
|---|---|
| 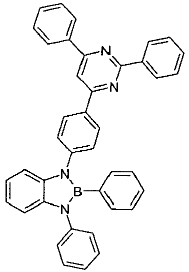 | 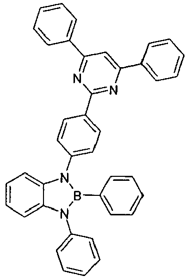 |
| 1 | 2 |
| 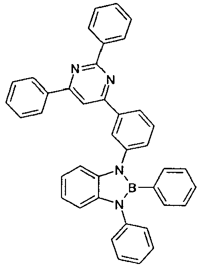 | 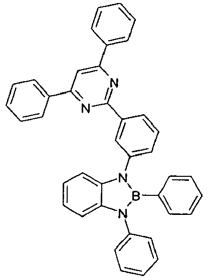 |
| 3 | 4 |
| 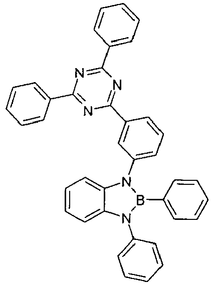 | 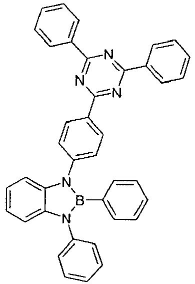 |
| 5 | 6 |
| 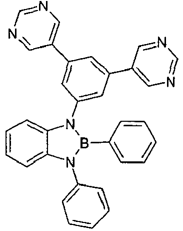 | 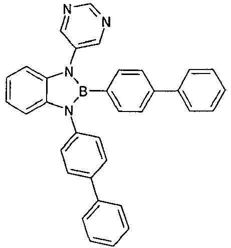 |
| 7 | 8 |
| 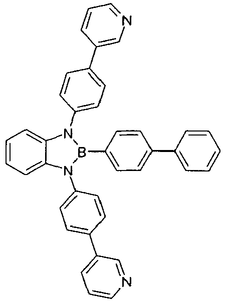 | 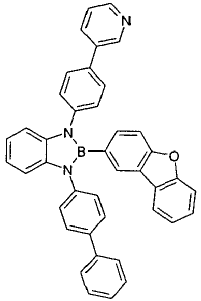 |
| 9 | 10 |
| 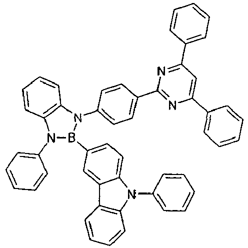 | 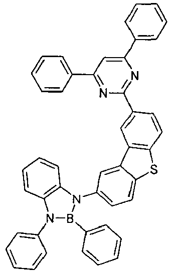 |
| 11 | 12 |
| 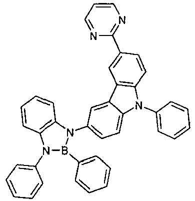 | 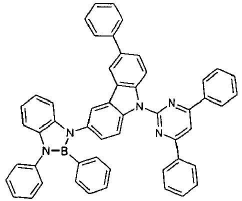 |
| 11 | 12 |
| 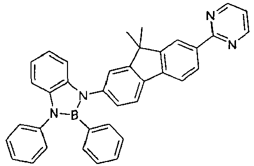 | 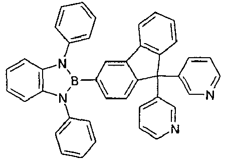 |
| 13 | 14 |
| 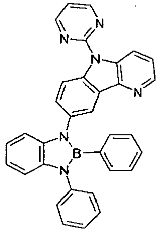 | 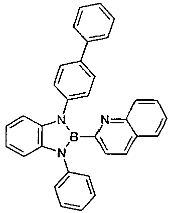 |
| 15 | 16 |
| 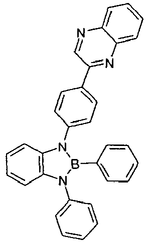 | 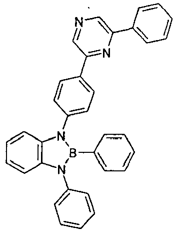 |
| 17 | 18 |
| 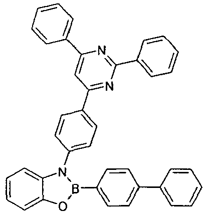 | 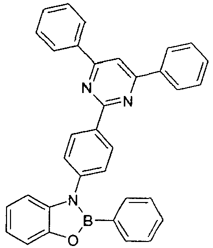 |
| 19 | 20 |
| 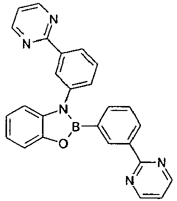 | 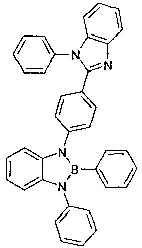 |
| 21 | 22 |
| 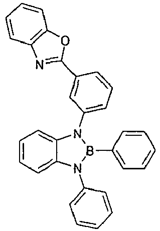 | 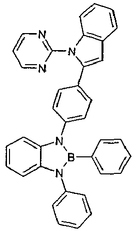 |
| 23 | 24 |
| 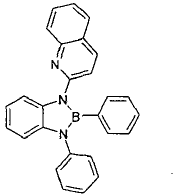 | 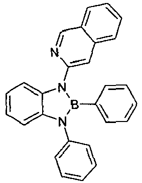 |
| 25 | 26 |
| 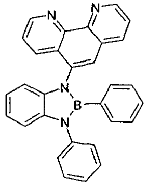 | 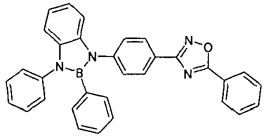 |
| 27 | 28 |
| 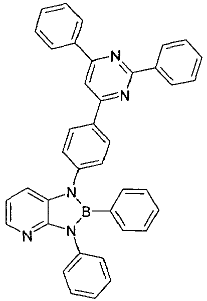 | 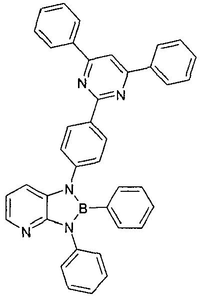 |
| 29 | 30 |
| 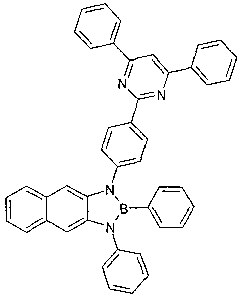 | 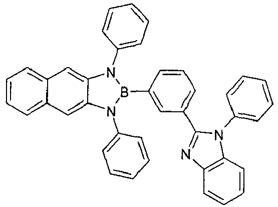 |
| 31 | 32 |
| 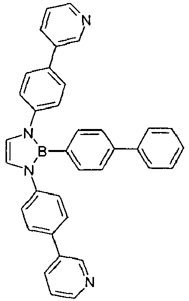 | 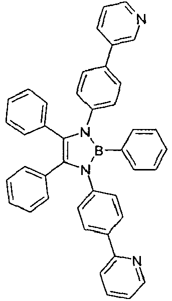 |
| 33 | 34 |
| 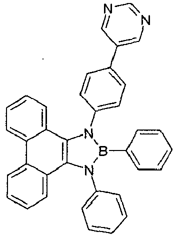 | 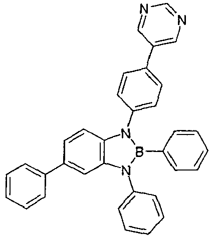 |
| 35 | 36 |
| 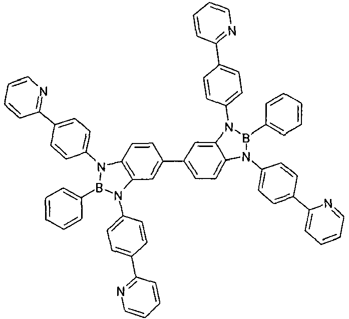 | 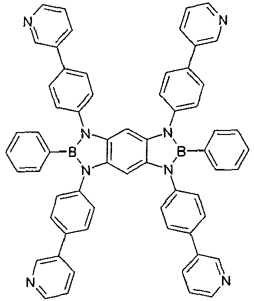 |
| 35 | 36 |
| 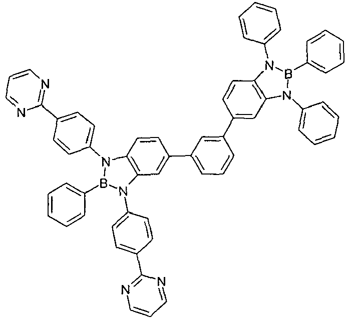 | 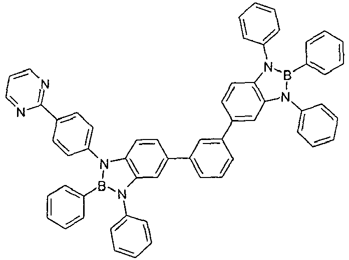 |
| 37 | 38 |
| 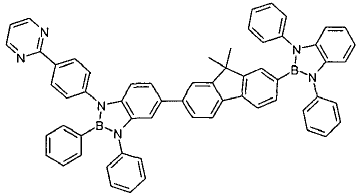 | 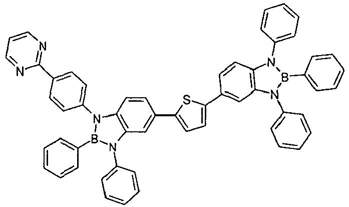 |
| 39 | 40 |
| 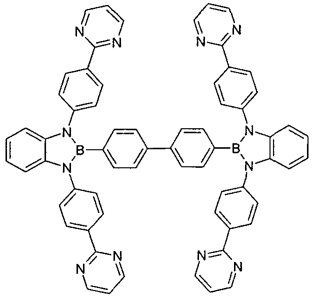 | 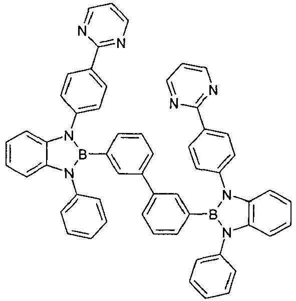 |
| 41 | 42 |
| 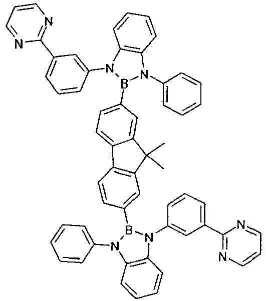 | 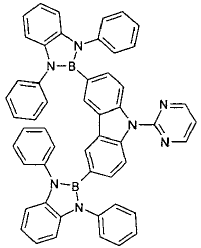 |
| 43 | 44 |
| 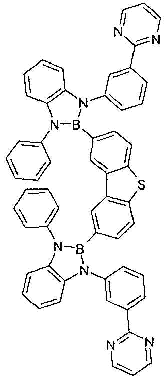 | 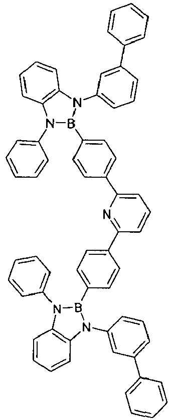 |
| 45 | 46 |
| 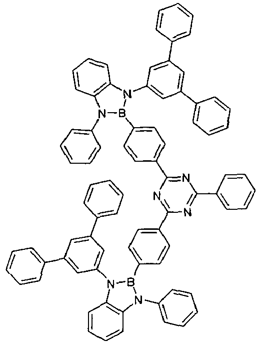 | 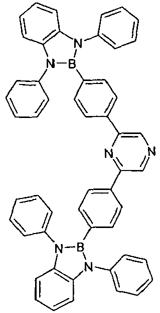 |
| 47 | 48 |
| 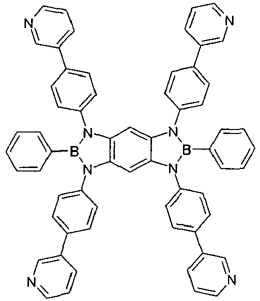 | 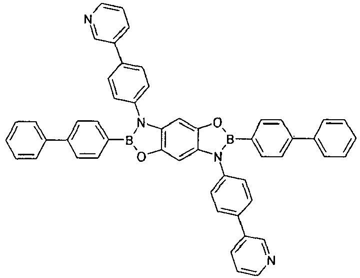 |
| 49 | 50 |

Zur Herstellung der Verbindungen gemäß Formel (1) bzw. der erfindungsgemäßen Verbindungen hat sich das im Folgenden beschriebene Verfahren als besonders gut geeignet herausgestellt. Die erfindungsgemäßen Verbindungen können durch bekannte organisch-chemische Syntheseverfahren hergestellt werden. Dazu zählen beispielsweise die Hartwig-Buchwald-Kupplung, die Suzuki-Kupplung sowie Cyclokondensationen mit Organoborverbindungen.

Das folgende **Schema 1** zeigt die Synthese der erfindungsgemäßen Verbindungen **A.**

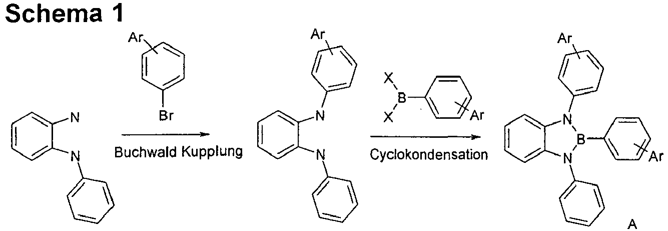

Zur Synthese des Grundkörpers **A** wird an N-Phenylbenzol-1,2-diamin zunächst in einer Buchwald-Reaktion ein Bromaryl gekuppelt. Die Umsetzung der resultierenden Verbindung mit Bor-Verbindungen, beispielsweise Bordibrombenzol, ergibt die entsprechenden Diazaborolin-Verbindungen.

**Schema 2** zeigt die Synthese des Grundkörpers **B.** Sie unterscheidet sich von der in Schema 1 gezeigten Synthese lediglich dadurch, dass statt Bordibromid-Verbindungen das 1,4-Bis(dibromborbenzol) in der Cyclokondensation mit zwei Äquivalenten des Diamins eingesetzt wird.

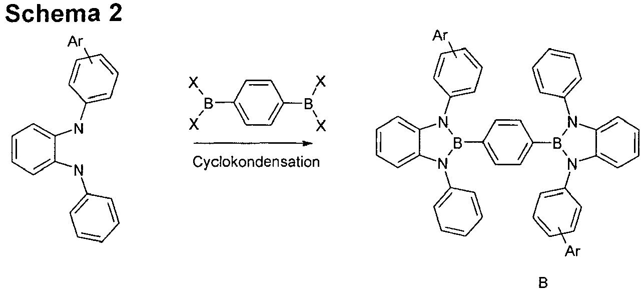

Für die Grundkörper **C, D** und **E** kann analog verfahren werden, wie in Schema 3 und 4 dargestellt.

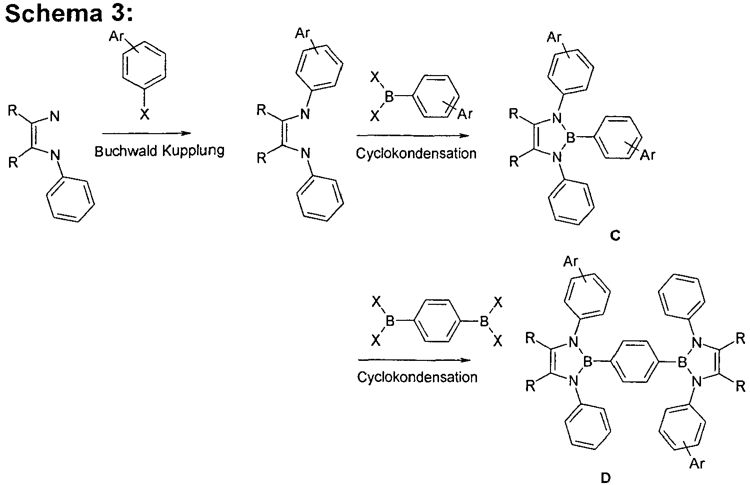

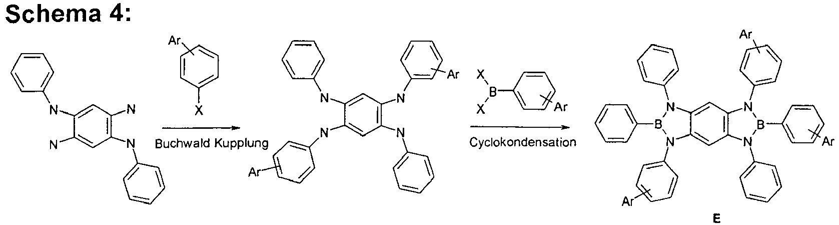

Die oben beschriebenen Synthesewege sollen lediglich als Beispiele dienen. Der Fachmann kann zur Synthese der erfindungsgemäßen Verbindungen auf alternative Syntheseverfahren zurückgreifen, wenn ihm dies unter den gegebenen Umständen vorteilhaft erscheint. Weiterhin kann er unter Heranziehung seines allgemeinen Fachwissens auf dem Gebiet der organischen Synthesechemie die gezeigten Synthesen erweitern und/oder modifizieren, um erfindungsgemäße Verbindungen herzustellen.

Ein weiterer Gegenstand der vorliegenden Erfindung ist ein Verfahren zur Herstellung der erfindungsgemäßen Verbindungen durch Umsetzung einer aromatischen ortho-Diaminoverbindung mit einer Aryl- bzw. Heteroarylborverbindung, in der das Boratom mit zwei reaktiven Abgangsgruppen, insbesondere Chlor oder Brom, substituiert ist.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird, insbesondere ein phosphoreszierender Dotand. Geeignete Dotanden sind unten im Zusammenhang mit den organischen Elektrolumineszenzvorrichtungen aufgeführt und sind auch für die erfindungsgemäßen Mischungen bevorzugt.

Für die Verarbeitung aus Lösung bzw. aus flüssiger Phase, beispielsweise durch Spin-Coating oder durch Druckverfahren, sind Lösungen bzw. Formulierungen der erfindungsgemäßen Verbindungen bzw. Mischungen erforderlich. Es kann bevorzugt sein, Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Formulierung, insbesondere eine Lösung, eine Suspension oder eine Miniemulsion, enthaltend mindestens eine erfindungsgemäße Verbindung oder Mischung und ein oder mehrere Lösemittel, insbesondere organische Lösemittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) oder gemäß den bevorzugten Ausführungsformen als Matrixmaterial für fluoreszierende oder phosphoreszierende Emitter, insbesondere für phosphoreszierende Emitter, und/oder in einer Elektronentransportschicht und/oder in einer elektronenblockierenden bzw. exzitonenblockierenden Schicht und/oder in einer Lochtransportschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand verstanden. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder gemäß der nicht offen gelegten Anmeldung EP 11003232.3, Triphenylenderivaten, z. B. gemäß der nicht offen gelegten Anmeldung DE 102010048608.6, oder Lactame, z. B. gemäß den nicht offen gelegten Anmeldungen DE 102010012738.8 oder DE 102010019306.2. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898 entnommen werden. Weiterhin eignen sich die Komplexe gemäß den nicht offen gelegten Anmeldungen EP 10006208.2 und DE 102010027317.1. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Elektronentransportschicht und/oder in einer Lochblockierschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. gemäß den bevorzugten Ausführungsformen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für fluoreszierende oder phosphoreszierende Emitter führen zu sehr hohen Effizienzen und/oder zu langen Lebensdauern. Dies gilt insbesondere, wenn die Verbindungen als Matrixmaterial für einen phosphoreszierenden Emitter eingesetzt werden. Dabei werden wesentlich bessere Effizienzen, insbesondere Leistungseffizienzen, und Lebensdauern erhalten als bei Einsatz von strukturell ähnlichen Boronsäurederivaten als Matrixmaterialien.
2. Die erfindungsgemäßen Verbindungen eignen sich nicht nur als Matrix für grün und rot phosphoreszierende Verbindungen, sondern je nach Struktur auch für blau phosphoreszierende Verbindungen.
3. Die erfindungsgemäßen Verbindungen weisen eine hohe thermische Stabilität auf.
4. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatzspannungen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Komplexe herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die Angaben in eckigen Klammern stellen jeweils die CAS-Nummer der literaturbekannten Edukte dar.

### Beispiel 1: 1-[4-(4,6-Diphenylpyrimidin-2-yl)-phenyl]-2,3-diphenyl-2,3-dihydro-1H-benzo[1,3,2]diazaborol

### a) 2-(4-Bromphenyl)-4,6-diphenyl-pyrimidin

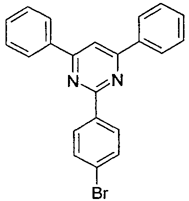

10.0 g (53.8 mmol) 4-Brombenzonitril und 11.5 g (200 mmol) Hydroxylammoniumchlorid werden unter Rühren bei Raumtemperatur in 400 mL Methanol gelöst. Anschließend werden 14.9 g (17.8 mmol) Natriumhydrogensulfat sowie 60 mL VE-Wasser zugegeben. Die Reaktionsmischung wird für 3 h unter Rückfluss gekocht. Die gelbe Reaktionslösung wird unter vermindertem Druck aufkonzentriert. Der verbleibende Rückstand wird aus Methanol umkristallisiert. Die Ausbeute beträgt 11.0 g von 4-Brom-N-hydroxy-benzamidin (76 % d. Th.).

53.2 g (0.3 mol) 4-Brom-N-hydroxy-benzamidin, 42.7g (0.2 mol) Benzylidenacetophenon und 0.6 mL (10,0 mmol) Eisessig werden suspendiert und für 30 Min bei Raumtemperatur gerührt. Anschließend wird der Ansatz 24 h bei 150 °C gerührt. Der entstandene Rückstand wird mit Toluol gewaschen und mittels präparativer Kieselgel-Chromatographie aufgereinigt. Ausbeute: 35.0 g (42.4 % d. Th.).

### b) (4,6-Diphenyl-pyrimidin-2-yl)-phenyl]-N'-phenyl-1,2-diaminobenzol

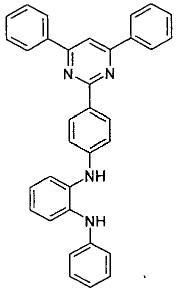

20.0 g (108.5 mmol) N-Phenyl-1,2-diaminobenzol, 44.0 g (0.11 mol) 2-(4-Bromphenyl)-4,6-diphenylpyrimidin, 4.4 g (5.4 mmol) 1,1-Bis(diphenyl-phosphino)ferrocen-dichloropalladium(II) Komplex mit Dichlormethan und 32.2 g (325 mmol) Natrium-tert-butylat werden in 300 mL Toluol 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 25.0 g (42 % d. Th).

### c) 4-(4,6-Diphenylpyrimidin-2-yl)-phenyl]-2,3-diphenyl-2,3-dihydro-1H-benzo[1,3,2]diazaborol

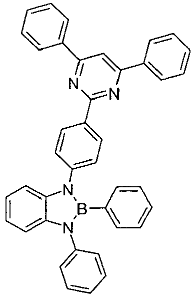

9.3 g (61 mmol) Trimethyl-phenyl-silan werden in 250 mL Toluol unter Rühren bei Raumtemperatur gelöst. Anschließend werden langsam 11.4 mL (118 mmol) Bortribromid zugetropft und die bräunliche Lösung für 2 h unter Rückfluss erhitzt. Nach beendeter Reaktion werden 150 mL getrocknetes Toluol zugegeben und per Wasserabscheider ca. 300 mL Toluol abdestilliert, um die überschüssige Menge BBr₃ zu entfernen. Dann wird der Ansatz auf ca. 60 mL Toluol aufkonzentriert und unter Schutzgas gelagert.

27.0 g (55.0 mmol) (4,6-Diphenyl-pyrimidin-2-yl)-phenyl]-N'-phenyl-1,2-diaminobenzol werden in 500 mL Toluol gelöst, 31.0 mL (220.1 mmol) Triethylamin zugegeben und anschließend auf 0 °C gekühlt. Zu der Reaktionmischung werden 61 mL (61 mmol) einer 1 M Lösung Dibrom-(phenyl)boran in Toluol unter Rühren bei 0 °C langsam getropft. Die Reaktionsmischung wird über einen Zeitraum von 30 Min. auf Raumtemperatur erwärmt. Nach Abkühlen der Reakionsmischung werden 600 mL EtOH zugetropft. Der ausgefallene Feststoff wird aus Toluol umkristallisiert und anschließend sublimiert 28.4 g (89.6 % d. Th, Reinheit > 99.9 %)

### Beispiel 2: 3-[4-(2,6-Diphenylpyrimidin-4-yl)phenyl]-1,2-diphenyl-1,3,2-benzodiazaborol

### a) 4-(4-Bromphenyl)-2,6-diphenylpyrimidin

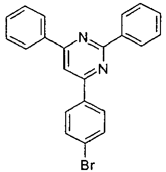

107.7 g (0.5 mol) 4-Bromacetophenon und 50.0 mL (0.5 mol) Benzaldehyd werden vorgelegt und mit 815 mL Natronlauge (2 mol/L, 1.6 mol) sowie 850 mL VE-Wasser versetzt. Das Reaktionsgemisch wird für 24 h bei 40 °C gerührt. Der ausgefallene Feststoff wird abgesaugt und mit VE-Wasser nachgewaschen. Das so erhaltene Rohprodukt wird aus Ethanol umkristallisiert. Ausbeute 113 g (80 % d. Th.).

21.8 g (0,4 mol) KOH-Plätzchen werden in 500 mL EtOH gelöst. Anschließend werden 38.0 g (0.2 mol) Benzamidin-Hydrochlorid und 115.0 g (0.4 mol) (E)-1-(4-Bromphenyl)-3-phenylprop-2-en-1-on, jeweils in 250 mL Ethanol gelöst, zugegeben und das Gemisch für 3 h unter Rückfluss erhitzt. Nach dem Abkühlen auf Raumtemperatur wird der ausgefallene Feststoff abgesaugt, mehrfach mit Ethanol gewaschen und getrocknet. Ausbeute: 76.0 g (81 % d. Th.).

### b) 3-[4-(2,6-Diphenylpyrimidin-4-yl)phenyl]-1,2-diphenyl-1,3,2-benzodiazaborol

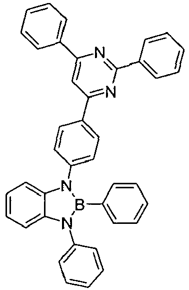

14.3 g N-Phenyl-1,2-diaminobenzol (77.5 mmol), 30.0 g (77.5 mol) 4-(4-Bromphenyl)-2,6-diphenylpyrimidin, 3.2 g (3.9 mmol) 1,1-Bis(diphenyl-phosphino)ferrocen-dichloropalladium(II) Komplex mit Dichlormethan, und 23 g Natrium-tert-butylat (232 mmol) werden in 300 mL Toluol 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 27.0 g (71 % d. Th).

23.6 g (48.0 mmol) des Diaminobenzol-Derivats werden in 500 mL Toluol gelöst, 27.1 mL (192 mmol) Triethylamin zugegeben und anschließend auf 0 °C gekühlt. Zu der Reaktionmischung werden 50 mL einer 1 M Lösung Dibrom-(phenyl)boran (50 mmol) in Toluol unter Rühren bei 0 °C langsam getropft. Die Reaktionsmischung wird über einen Zeitraum von 30 Min. auf Raumtemperatur erwärmt. Nach Abkühlung der Reakionsmischung werden 600 mL Ethanol zugetropft. Der ausgefallene Feststoff wird aus Toluol umkristallisiert und anschließend sublimiert. Ausbeute: 20.2 g (73 % d. Th., Reinheit > 99.9 %).

### Beispiel 3: 2-Biphenyl-4-yl-3-[4-(4,6-diphenyl-pyrimidin-2-yl)-phenyl]-2,3-dihydro-benzo[1,3,2]oxazaborol

### a) 2-[4-(4,6-Diphenyl-pyrimidin-2-yl)-phenylamino]-phenol

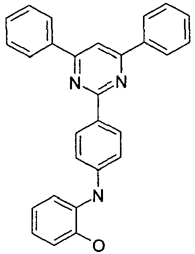

10.0 g 2-Aminophenol (137 mmol), 53 g (137 mmol) 2-(4-Bromphenyl)-4,6-diphenylpyrimidin, 5.3 g (27.4 mmol) Cul und 89 g (274 mmol) Cs₂CO₃ werden in 300 mL DMF 5 h unter Schutzatmosphäre zum Sieden erhitzt. Das Gemisch wird im Anschluss zwischen Essigester und Wasser verteilt, die organische Phase dreimal mit Wasser gewaschen und über Na₂SO₄ getrocknet und einrotiert. Der verbleibende Rückstand wird aus Heptan/ Toluol umkristallisiert. Die Ausbeute beträgt 39.9 g (70 % d. Th).

### b) 2-Biphenyl-4-yl-3-[4-(4,6-diphenyl-pyrimidin-2-yl)-phenyl]-2,3-dihydro-benzo[1,3,2]oxazaborol

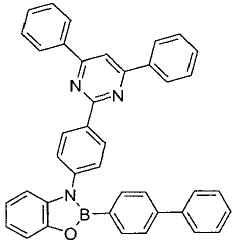

25.6 g (62.0 mmol) des Aminobenzol-Derivats werden in 500 mL Toluol gelöst, 26 mL (185 mmol) Triethylamin zugegeben und anschließend auf 0 °C gekühlt. Zu der Reaktionmischung werden 62 mL einer 1 M Lösung Dibrom-(4-biphenyl)boran (62 mmol) in Toluol unter Rühren bei 0 °C langsam getropft. Die Reaktionsmischung wird über einen Zeitraum von 30 Min. auf Raumtemperatur erwärmt. Nach Abkühlung der Reakionsmischung werden 600 mL Ethanol zugetropft. Der ausgefallene Feststoff wird aus Toluol umkristallisiert und anschließend sublimiert. Ausbeute: 17.8 g (50 % d. Th., Reinheit > 99.9 %).

### Beispiel 4: 3,3'-Bis-(1-phenyl-3-(4-pyrimidin-2-yl-phenyl)-2,3-dihydro-1H-benzo[1,3,2]diazaborol-2-yl)-biphenyl

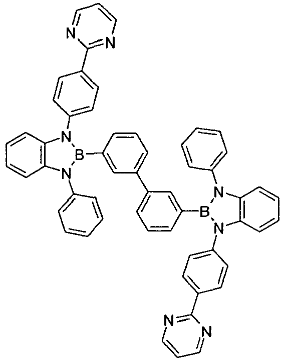

40.0 g (217 mmol) N-Phenyl-1,2-diaminobenzol, 51 g (217 mmol) 2-(4-Bromphenyl)pyrimidin, 8.8 g (10.9 mmol) 1,1-Bis(diphenylphosphino)-ferrocen-dichloropalladium(II) Komplex mit Dichlormethan, und 64.5 g Natrium-tert-butylat (651 mmol) werden in 600 mL Toluol 8 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 44.1 g (60 % d. Th).

30 g (89.0 mmol) (2-Pyrimidinyl)-phenyl]-N'-phenyl-1,2-diaminobenzol werden in 600 mL Toluol gelöst, 62 mL (443 mmol) Triethylamin zugegeben und anschließend auf 0 °C gekühlt. Zu der Reaktionmischung werden 45 mL einer 1M Lösung Bis(dibrom-(3,3'-diphenyl)boran) (45 mmol) in Toluol unter Rühren bei 0 °C langsam getropft. Die Reaktionsmischung wird über einen Zeitraum von 30 Min. auf Raumtemperatur erwärmt. Nach Abkühlung der Reakionsmischung werden 600 mL Ethanol zugetropft. Der ausgefallene Feststoff wird aus Toluol umkristallisiert und anschließend sublimiert. Ausbeute: 37 g (70 % d. Th, Reinheit > 99.9 %)

### Beispiel 5: 1-[4-(4,6-Diphenyl-pyrimidin-2-yl)-phenyl]-2,3-diphenyl-2,3-dihydro-1H-[1,3,2]diazaborolo[4,5-b]pyridin

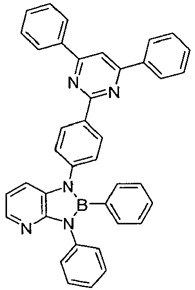

15.0 g (81 mmol) N-Phenylpyridin-2,3-diamin, 31.5 g (81 mmol) 2-(4-Bromphenyl)pyrimidin, 3.3 g (4 mmol) 1,1-Bis(diphenylphosphino)ferrocen-di-chloropalladium(II) Komplex mit Dichlormethan, und 23.3 g Natrium-tert-butylat (243 mmol) werden in 400 mL Toluol 8 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 17 g (45 % d. Th).

17 g (34.6 mmol) des Diaminopyridin-Derivats werden in 200 mL Toluol gelöst, 19.5 mL (138 mmol) Triethylamin zugegeben und anschließend auf 0 °C gekühlt. Zu der Reaktionmischung werden 38 mL einer 1 M Lösung Dibrom(phenyl)boran (38 mmol) in Toluol unter Rühren bei 0 °C langsam getropft. Die Reaktionsmischung wird über einen Zeitraum von 30 Min. auf Raumtemperatur erwärmt. Nach Abkühlung der Reakionsmischung werden 200 mL Etthanol zugetropft. Der ausgefallene Feststoff wird aus Toluol umkristallisiert und anschließend sublimiert. Ausbeute: 12 g (60 % d. Th., Reinheit > 99.9 %).

### Beispiel 6: 2-[4-(2,6-Diphenyl-pyrimidin-4-yl)-phenyl]-1,3-diphenyl-2,3-dihydro-1H-benzo[1,3,2]diazaborol

### a) 2-Brom-1,3-diphenyl-2,3-dihydro-1H-benzo[1,3,2]diazaborol

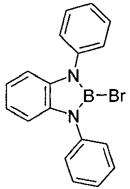

20.0 g (77 mmol) N,N'-Diphenyl-benzol-1,2-diamin werden in 300 mL Toluol gelöst, 42 mL (307 mmol) Triethylamin zugegeben und anschließend auf 0 °C gekühlt. Zu der Reaktionmischung werden 8.14 mL (85 mmol) Bortribromid in 20 mL Toluol unter Rühren bei 0 °C langsam getropft. Die Reaktionsmischung wird über einen Zeitraum von 30 Min auf Raumtemperatur erwärmt. Nach beendeter Reaktion werden 200 mL getrocknetes Toluol zugegeben und per Wasserabscheider ca. 400 mL Toluol abdestilliert, um die überschüssige Menge BBr₃ zu entfernen. Dann wird der Ansatz auf ca. 100 mL aufkonzentriert und unter Schutzgas gelagert.

### b) 2-[4-(2,6-Diphenyl-pyrimidin-4-yl)-phenyl]-1,3-diphenyl-2,3-dihydro-1H-benzo[1,3,2]diazaborol

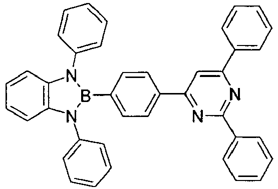

11.9 g (34 mmol) 4-(4-Bromphenyl)-2,6-diphenylpyrimidin werden in 300 mL THF gelöst und anschließend auf -100 °C gekühlt. Zu der Reaktionsmischung werden 22 mL (38 mmol) einer 1.6M Lösung n-Butyllithium in Hexan unter Rühren langsam getropft. Die Reaktionsmischung wird 30 Min. gerührt. Anschließend werden 200 mL (34 mmol) einer Lösung von 2-Brom-1,3-diphenyl-2,3-dihydro-1H-benzo[1,3,2]diazaborol in Toluol zugetropft. Die Reakionsmischung wird über einen Zeitraum von 4 h auf 0 °C erwärmt, dann werden 600 mL Ethanol zugetropft. Der ausgefallene Feststoff wird aus Toluol umkristallisiert und anschließend sublimiert. Ausbeute: 11 g (60 % d. Th, Reinheit > 99.9 %).

### Beispiel 7: Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1, E1-E12 (siehe Tabellen 1 und 2) werden die Daten verschiedener OLEDs vorgestellt. Glasplättchen, die mit strukturiertem ITO (Indium-Zinn-Oxid) der Dicke 50 nm beschichtet sind werden zur verbesserten Prozessierung mit 20 nm PEDOT:PSS beschichtet (Poly-(3,4-ethylendioxythiophen)poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden. Die OLEDs haben prinzipiell folgenden Schichtaufbau: Substrat / Lochtransportschicht (HTL) / Zwischenschicht (IL) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie DAB1:VCbz1:TEG1 (65%:30%:5%) bedeutet hierbei, dass das Material DAB1 in einem Volumenanteil von 65%, VCbz1in einem Anteil von 30% und TEG1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt.

### Verwendung von erfindungsgemäßen Verbindungen als Matrixmaterialien in phosphoreszierenden OLEDs

Verwendet man erfindungsgemäße Materialien als Matrix für phosphoreszierende grüne Dotanden, so erhält man sehr gute Effizienzen. Mit der Verbindung DAB1 z. B. erreicht man eine externe Quanteneffizinz von 18.3% und eine Leistungseffizienz von 52 Im/W (Beispiel E1). In Mischung mit dem Dimer eines verbrückten Carbazols VCbz1 erreicht man sogar 19.2% und 59 Im/W (Beispiel E3). Weiterhin erhält man mit erfindungsgemäßen Verbindungen gute Lebensdauern. Wird z. B. die OLED aus Beispiel E1 bei einer Stromdichte von 20 mA/cm² betrieben, so fällt die Leuchtdichte innerhalb von 120 h auf 70% ihres Anfangswertes ab.

Verwendet man das Diazaborol DAB7, welches nicht mit einer heteroaromatischen Gruppe substituiert ist, so erhält man höhere Betriebsspannungen sowie geringere Effizienzen als mit den vergleichbaren erfindungsgemäßen Verbindungen DAB1 und DAB2 (Beispiel V1).

**Tabelle 1: Aufbau der OLEDs**

| Bsp. | HTL Dicke | IL Dicke | EBL Dicke | EML Dicke | HBL Dicke | ETL Dicke | EIL Dicke |
|---|---|---|---|---|---|---|---|
| V1 | SpA1 | HATCN | SpMA1 | DAB7:TEG1 | IC1 | ST1:LiQ (50%:50%) | --- |
| | 70nm | 5nm | 90nm | (90%:10%) 30nm | 10nm | 30nm | |
| E1 | SpA1 | HATCN | SpMA1 | DAB1:TEG1 | IC1 | ST1:LiQ (50%:50%) | --- |
| | 70nm | 5nm | 90nm | (90%:10%) 30nm | 10nm | 30nm | |
| E2 | SpA1 | HATCN | SpMA1 | DAB1:TEG1 | --- | ST1:LiQ (50%:50%) | --- |
| | 70nm | 5nm | 90nm | (90%:10%) 30nm | | 40nm | |
| E3 | SpA1 | HATCN | SpMA1 | DAB1:VCbz1:TEG1 | IC1 | ST1:LiQ (50%:50%) | --- |
| | 70nm | 5nm | 90nm | (65%:30%:5%) 30nm | 10nm | 30nm | |
| E4 | SpA1 | HATCN | SpMA1 | DAB2:TEG1 | IC1 | ST1:LiQ (50%:50%) | --- |
| | 70nm | 5nm | 90nm | (90%:10%) 30nm | 10nm | 30nm | |
| E5 | SpA1 | HATCN | SpMA1 | DAB2:TEG1 | --- | ST1:LiQ (50%:50%) | --- |
| | 70nm | 5nm | 90nm | (90%:10%) 30nm | | 40nm | |
| E6 | SpA1 | HATCN | SpMA1 | DAB2:VCbz1:TEG1 | IC1 | ST1:LiQ(50%:50%) | --- |
| | 70nm | 5nm | 90nm | (65%:30%:5%) 30nm | 10nm | 30nm | |
| E7 | SpA1 | HATCN | SpMA1 | DAB3:TEG1 | IC1 | ST1:LiQ (50%:50%) | --- |
| | 70nm | 5nm | 90nm | (90%:10%) 30nm | 10nm | 30nm | |
| E8 | SpA1 | HATCN | SpMA1 | DAB4:TEG1 | IC1 | ST1:LiQ (50%:50%) | --- |
| | 70nm | 5nm | 90nm | (90%:10%) 30nm | 10nm | 30nm | |
| E9 | SpA1 | HATCN | SpMA1 | DAB5:TEG1 | IC1 | ST1:LiQ (50%:50%) | --- |
| | 70nm | 5nm | 90nm | (90%:10%) 30nm | 10nm | 30nm | |
| E10 | SpA1 | HATCN | SpMA1 | DAB6:TEG1 | IC1 | ST1:LiQ (50%:50%) | --- |
| | 70nm | 5nm | 90nm | (90%:10%) 30nm | 10nm | 30nm | |
| E11 | SpA1 | HATCN | SpMA1 | DAB6:VCbz1:TEG1 | IC1 | ST1:LiQ (50%:50%) | --- |
| | 70nm | 5nm | 90nm | (65%:30%:5%) 30nm | 10nm | 30nm | |
| E12 | SpA1 | HATCN | SpMA1 | IC1:TEG1 | --- | DAB1 | LiQ |
| | 70nm | 5nm | 90nm | (90%:10%) 30nm | | 40nm | 3nm |

**Tabelle 2: Daten der OLEDs**

| Bsp. | U1000 (V) | SE1000 (cd/A) | LE1000 (lm/W) | EQE 1000 | CIE x/y bei 1000 cd/m² |
|---|---|---|---|---|---|
| V1 | 4.9 | 37 | 24 | 9.8% | 0.34/0.64 |
| E1 | 3.9 | 66 | 52 | 18.3% | 0.33/0.63 |
| E2 | 3.6 | 58 | 51 | 16.5% | 0.33/0.63 |
| E3 | 3.7 | 70 | 59 | 19.2% | 0.32/0.63 |
| E4 | 4.3 | 62 | 45 | 17.1% | 0.33/0.63 |
| E5 | 4.1 | 59 | 45 | 16.4% | 0.33/0.63 |
| E6 | 3.8 | 64 | 52 | 17.6% | 0.32/0.63 |
| E7 | 3.5 | 61 | 55 | 17.0% | 0.33/0.63 |
| E8 | 4.2 | 60 | 44 | 16.6% | 0.33/0.63 |
| E9 | 3.8 | 63 | 52 | 17.4% | 0.33/0.63 |
| E10 | 4.1 | 60 | 46 | 16.8% | 0.33/0.63 |
| E11 | 3.9 | 64 | 52 | 17.9% | 0.32/0.63 |
| E12 | 4.0 | 59 | 46 | 16.3% | 0.33/0.63 |

**Tabelle 3: Strukturformeln der Materialien für die OLEDs**

| | |
|---|---|
| 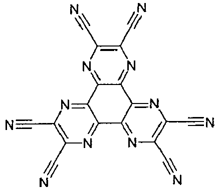 | 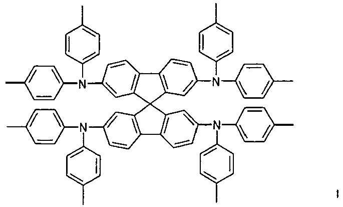 |
| HATCN | SpA1 |
| 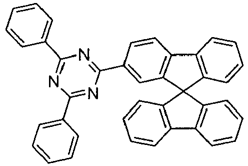 | 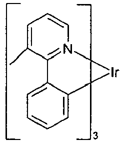 |
| ST1 | TEG1 |
| 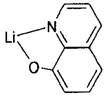 | 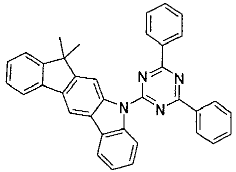 |
| LiQ | IC1 |
| 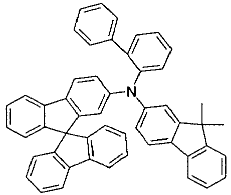 | 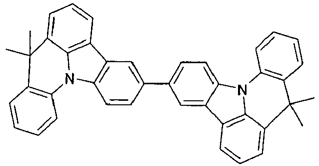 |
| SpMA1 | VCbz1 |
| 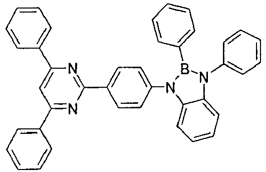 | 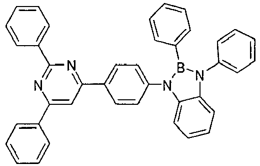 |
| DAB1 | DAB2 |
| 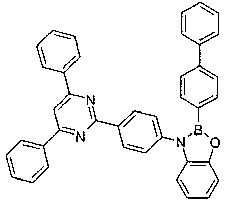 | 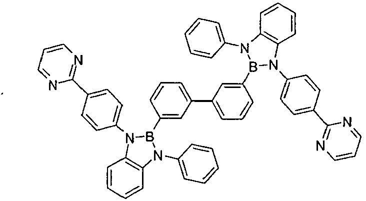 |
| DAB3 | DAB4 |
| 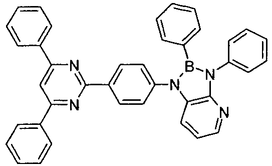 | 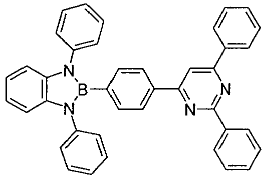 |
| DAB5 | DAB6 |
| 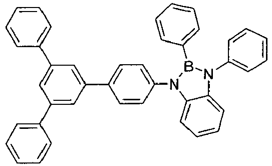 | |
| DAB7 (Stand der Technik) | |

## Patentansprüche

1. Verbindung gemäß Formel (1), 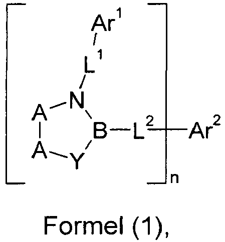 wobei für die verwendeten Symbole und Indizes gilt:
A-A ist gleich oder verschieden bei jedem Auftreten eine Einheit der gemäß Formel (2), (3), (4), (5), (6), (7) oder (8), 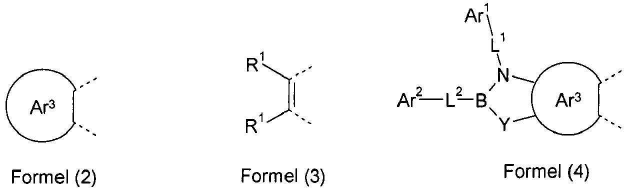 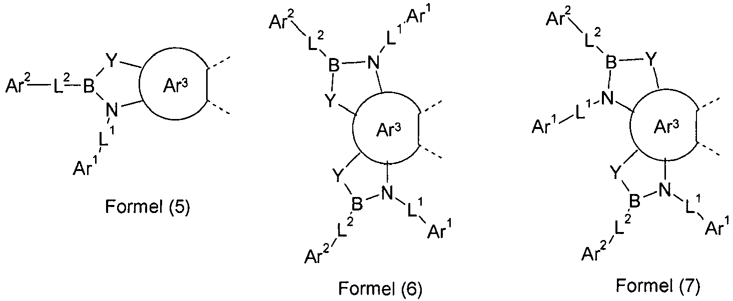 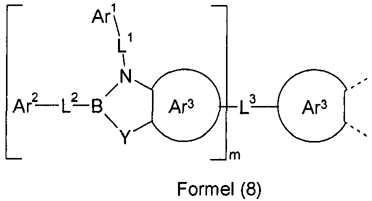 wobei die gestrichelte Bindung jeweils die Verknüpfung mit N bzw. Y darstellt;
Y ist gleich oder verschieden bei jedem Auftreten N-R², O oder S;
Ar¹, Ar² ist bei jedem Auftreten gleich oder verschieden eine Aryl- oder Heteroarylgruppe mit 5 bis 18 aromatischen Ringatomen, welche durch einen oder mehrere Reste R¹ substituiert sein kann; mit der Maßgabe, dass die Heteroatome aus N, O und/oder S ausgewählt sind, wenn Ar¹ oder Ar² eine Heteroarylgruppe ist;
Ar³ ist bei jedem Auftreten gleich oder verschieden eine Aryl- oder Heteroarylgruppe mit 5 bis 14 aromatischen Ringatomen, welche durch einen oder mehrere Reste R¹ substituiert sein kann;
L¹, L² ist bei jedem Auftreten gleich oder verschieden eine Einfachbindung oder ein bivalentes aromatisches oder heteroaromatisches Ringsystem mit 5 bis 24 aromatischen Ringatomen, das durch einen oder mehrere Reste R¹ substituiert sein kann;
L³ ist eine Einfachbindung oder eine bivalente, trivalente oder tetravalente Gruppe;
R¹ ist bei jedem Auftreten gleich oder verschieden ausgewählt aus der Gruppe bestehend aus H, D, F, Cl, Br, I, CN, NO₂, N(R³)₂, C(=O)R³, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C≡C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
R² ist bei jedem Auftreten gleich oder verschieden ausgewählt aus der Gruppe bestehend aus einer geradkettigen Alkylgruppe mit 1 bis 40 C-Atomen oder einer verzweigten oder cyclischen Alkyl-gruppe mit 3 bis 40 C-Atomen, die jeweils mit einem oder mehreren Resten R³ substituiert sein kann, wobei eine oder mehrere nicht-benachbarte CH₂-Gruppen durch R³C=CR³, C≡C oder C=O ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, oder einer Kombination dieser Systeme; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein;
R³ ist ausgewählt aus der Gruppe bestehend aus H, D, F, CN, aliphatischem Kohlenwasserstoffrest mit 1 bis 20 C-Atomen, aromatischem oder heteroaromatischem Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches, aromatisches oder heteroaromatisches Ringsystem bilden können;
n ist 1, 2, 3, 4, 5 oder 6;
m ist 1, wenn L³ eine Einfachbindung oder eine bivalente Gruppe ist, oder ist 2, wenn L³ eine trivalente Gruppe ist, oder ist 3, wenn L³ eine tetravalente Gruppe ist;
**dadurch gekennzeichnet, dass** mindestens eine Gruppe Ar¹ vorhanden ist, die für eine Heteroarylgruppe mit 5 bis 18 aromatischen Ringatomen steht, welche auch durch einen oder mehrere Reste R¹ substituiert sein kann, oder dass mindestens eine Gruppe Ar² vorhanden ist, die für eine elektronenarme Heteroarylgruppe mit 5 bis 18 aromatischen Ringatomen steht, wobei
eine elektronenarme Heteroarylgruppe entweder eine 5-Ring-Heteroarylgruppe mit mindestens zwei Heteroatomen oder eine 6-Ring-Heteroarylgruppe mit mindestens einem Heteroatom ist, wobei an diese Gruppen weitere 6-Ring-Aryl- oder 6-Ring-Heteroarylgruppen ankondensiert sein können.

2. Verbindung nach Anspruch 1, **dadurch gekennzeichnet, dass** Ar³ gleich oder verschieden bei jedem Auftreten für eine Aryl- oder Heteroarylgruppe mit 5 bis 10 aromatischen Ringatomen steht, welche jeweils durch einen oder mehrere Reste R¹ substituiert sein kann, bevorzugt ausgewählt aus der Gruppe bestehend aus Benzol, Pyridin, Pyrimidin, Pyridazin, Pyrazin, Furan, Thiophen, Pyrrol, Naphthalin, Phenanthren, Chinolin, Isochinolin, Chinoxalin, Indol, Benzofuran und Benzothiophen, welche jeweils durch einen oder mehrere Reste R¹ substituiert sein können.

3. Verbindung nach Anspruch 1 oder 2, ausgewählt aus den Verbindungen der Formeln (9) bis (18), 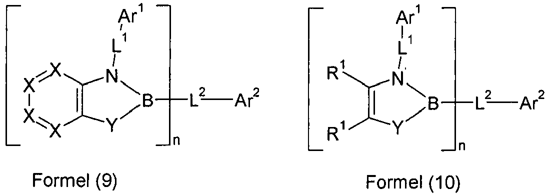 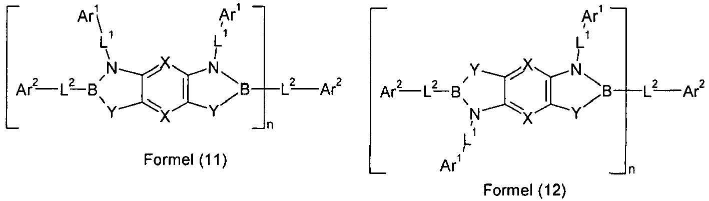 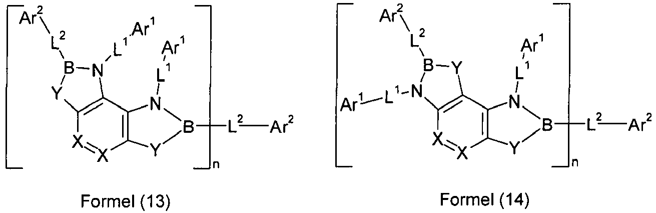 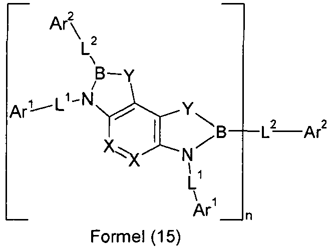 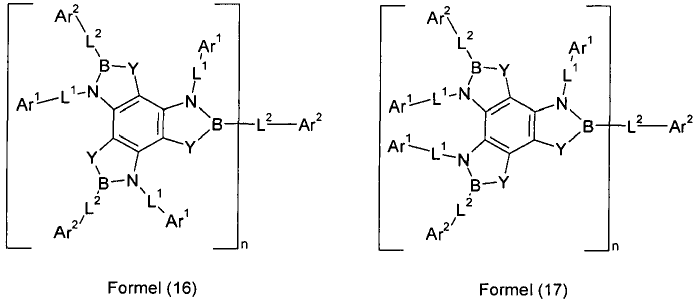 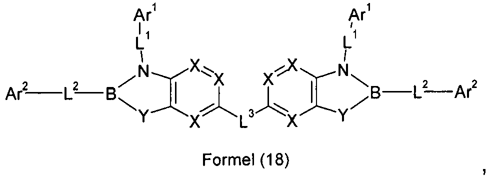 wobei X bei jedem Auftreten gleich oder verschieden CR¹ oder N ist und die weiteren die verwendeten Symbole und Indizes die in Anspruch 1 genannten Bedeutungen aufweisen.

4. Verbindung nach einem oder mehreren der Ansprüche 1 bis 3,
**dadurch gekennzeichnet, dass** Y gleich oder verschieden bei jedem Auftreten für N-R² steht.

5. Verbindung nach einem oder mehreren der Ansprüche 1 bis 4,
**dadurch gekennzeichnet, dass** L¹ bzw. L² gleich oder verschieden bei jedem Auftreten für eine Einfachbindung oder eine bivalente Arylen- oder Heteroarylengruppe mit 5 bis 10 aromatischen Ringatomen, die jeweils durch einen oder mehrere Reste R¹ substituiert sein kann, steht, bevorzugt für 1,2-, 1,3-der 1,4-Phenylen, Pyridin, Pyrimidin oder Triazin, welches jeweils durch einen oder mehrere Reste R¹ substituiert sein kann.

6. Verbindung nach einem oder mehreren der Ansprüche 1 bis 5,
**dadurch gekennzeichnet, dass** Ar¹ bzw. Ar² aufgebaut sind aus jeweils einer oder mehreren Gruppen ausgewählt aus Benzol, Pyridin, Pyrimidin, Pyridazin, Pyrazin, Triazin, Pyrrol, Thiophen, Furan, Imidazol, Indol, Benzothiophen, Benzofuran, Benzimidazol, Carbazol, Dibenzofuran oder Dibenzothiophen, welche jeweils mit einem oder mehreren Resten R¹ substituiert sein können.

7. Verbindung nach einem oder mehreren der Ansprüche 1 bis 6,
**dadurch gekennzeichnet, dass** R² bei jedem Auftreten gleich oder verschieden ausgewählt ist aus der Gruppe bestehend aus einem aromatischen oder heteroaromatischen Ringsystem mit 5 bis 30 aromatischen Ringatomen, das jeweils mit einem oder mehreren Resten R³ substituiert sein kann; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein kann, bevorzugt Phenyl, Naphthyl, Biphenyl, Terphenyl oder Quaterphenyl, welches jeweils mit einem oder mehreren Resten R³ substituiert sein kann.

8. Verbindung nach einem oder mehreren der Ansprüche 3 bis 7,
**dadurch gekennzeichnet, dass** R³ wie in Anspruch 1 definiert ist und weiterhin gilt:
Y ist gleich oder verschieden bei jedem Auftreten für N-R²;
L¹, L² ist gleich oder verschieden bei jedem Auftreten eine Einfachbindung oder eine bivalente Arylen- oder Heteroarylengruppe mit 5 bis 10 aromatischen Ringatomen, die jeweils durch einen oder mehrere Reste R¹ substituiert sein kann.
L³ ist eine Einfachbindung, O, S, NR², eine Alkylengruppe mit 1 bis 10 C-Atomen, welche mit einem oder mehreren Resten R³ substituiert sein kann, oder ein aromatisches oder heteroaromatisches Ringsystem mit 5 bis 30 aromatischen Ringatomen, welches mit einem oder mehreren Resten R³ substituiert sein kann;
X ist bei jedem Auftreten gleich oder verschieden CR¹ oder N, wobei in jedem Cyclus maximal zwei Symbole X für N stehen und die anderen Symbole X gleich oder verschieden bei jedem Auftreten für CR¹ stehen;
Ar¹, Ar² steht gleich oder verschieden bei jedem Auftreten für ein aromatisches oder heteroaromatisches Ringsystem mit 5 bis 24 aromatischen Ringatomen, besonders bevorzugt mit 5 bis 18 aromatischen Ringatomen, wobei das aromatische oder heteroaromatische Ringsystem jeweils auch durch einen oder mehrere Reste R¹ substituiert sein kann; dabei ist mindestens eine Gruppe Ar¹ eine Heteroarylgruppe bzw. mindestens eine Gruppe Ar² eine elektronenarme Heteroarylgruppe, die durch einen oder mehrere Reste R¹ substituiert sein kann; mit der Maßgabe, dass die Heteroatome aus N, O und/oder S ausgewählt sind, wenn Ar¹ oder Ar² eine Heteroarylgruppe ist;
n ist 1, 2, 3 oder 4, bevorzugt 1, 2 oder 3;
R¹ ist gleich oder verschieden bei jedem Auftreten ausgewählt aus der Gruppe bestehend aus H, D, F, CN, N(R³)₂, einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen oder einer Alkenylgruppe mit 2 bis 20 C-Atomen, die jeweils mit einem oder mehreren Resten R³ substituiert sein kann, wobei eine oder mehrere nicht-benachbarte CH₂-Gruppen durch R³C=CR³ oder O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R³ substituiert sein kann, einer Aryloxy- oder Heteroaryloxygruppe mit 5 bis 30 aromatischen Ringatomen, die mit einem oder mehreren Resten R³ substituiert sein können, oder einer Kombination dieser Systeme, wobei optional zwei oder mehr benachbarte Substituenten R¹ ein monocyclisches oder polycyclisches, aliphatisches, aromatisches oder heteroaromatisches Ringsystem bilden können, das mit einem oder mehreren Resten R³ substituiert sein kann;
R² ist bei jedem Auftreten gleich oder verschieden ausgewählt aus der Gruppe bestehend aus einem aromatischen oder heteroaromatischen Ringsystem mit 5 bis 30 aromatischen Ringatomen, das jeweils mit einem oder mehreren Resten R³ substituiert sein kann; dabei können optional solche R¹ und R², die in 1,2-Position zueinander benachbart stehen, ein monocyclisches oder polycyclisches, aliphatisches, aromatisches oder heteroaromatisches Ringsystem bilden, das mit einem oder mehreren Resten R³ substituiert sein kann.

9. Mischung enthaltend mindestens eine Verbindung nach einem oder mehreren der Ansprüche 1 bis 8 und mindestens eine weitere Verbindung.

10. Formulierung enthaltend mindestens eine Verbindung oder Mischung nach einem oder mehreren der Ansprüche 1 bis 9 und ein oder mehrere Lösemittel.

11. Verfahren zur Herstellung einer Verbindung nach einem oder mehreren der Ansprüche 1 bis 8 durch Umsetzung einer aromatischen ortho-Diaminoverbindung mit einer Aryl- bzw. Heteroarylborverbindung, in der das Boratom mit zwei reaktiven Abgangsgruppen substituiert ist.

12. Verwendung einer Verbindung oder Mischung nach einem oder mehreren der Ansprüche 1 bis 9 in einer elektronischen Vorrichtung.

13. Elektronische Vorrichtung, enthaltend mindestens eine Verbindung oder Mischung nach einem oder mehreren der Ansprüche 1 bis 9, bevorzugt ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoff-sensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices".

14. Elektronische Vorrichtung nach Anspruch 13, wobei es sich um eine organische Elektrolumineszenzvorrichtung handelt, **dadurch gekennzeichnet, dass** die Verbindung nach einem oder mehreren der Ansprüche 1 bis 8 als Matrixmaterial für fluoreszierende oder phosphoreszierende Emitter und/oder in einer Elektronentransportschicht und/oder in einer Lochblockierschicht und/oder in einer elektronenblockierenden bzw. exzitonenblockierenden Schicht und/oder in einer Lochtransportschicht eingesetzt wird.

## Claims

1. Compound of the formula (1), 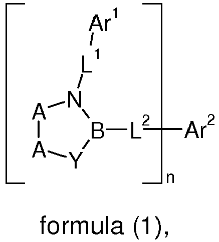 where the following applies to the symbols and indices used:
A-A is, identically or differently on each occurrence, a unit of the formula (2), (3), (4), (5), (6), (7) or (8), 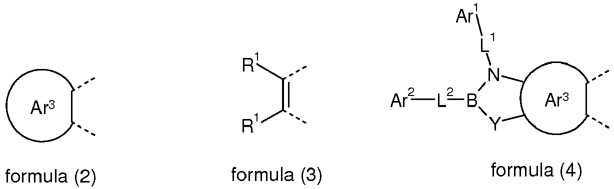 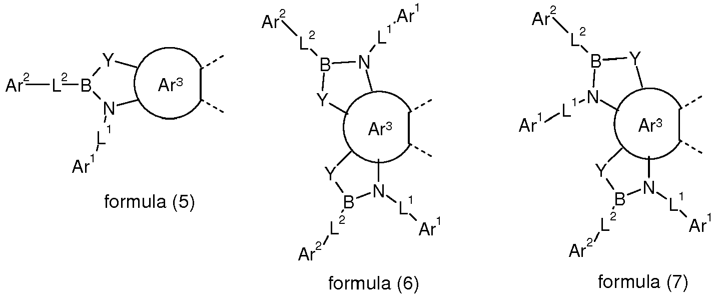 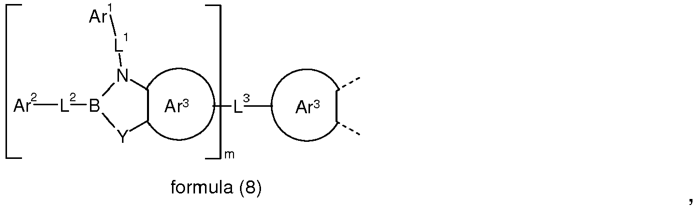 where the dashed bond in each case represents the link to N or Y;
Y is, identically or differently on each occurrence, N-R², O or S;
Ar¹, Ar² are on each occurrence, identically or differently, an aryl or heteroaryl group having 5 to 18 aromatic ring atoms, which may be substituted by one or more radicals R¹; with the proviso that the heteroatoms are selected from N, O and/or S if Ar¹ or Ar² is a heteroaryl group;
Ar³ is on each occurrence, identically or differently, an aryl or heteroaryl group having 5 to 14 aromatic ring atoms, which may be substituted by one or more radicals R¹;
L¹, L² are on each occurrence, identically or differently, a single bond or a divalent aromatic or heteroaromatic ring system having 5 to 24 aromatic ring atoms, which may be substituted by one or more radicals R¹;
L³ is a single bond or a divalent, trivalent or tetravalent group;
R¹ is selected on each occurrence, identically or differently, from the group consisting of H, D, F, Cl, Br, I, CN, NO₂, N(R³)₂, C(=O)R³, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, each of which may be substituted by one or more radicals R³, where one or more non-adjacent CH₂ groups may be replaced by R³C=CR³, C≡C, Si(R³)₂, Ge(R³)2, Sn(R³)2, C=O, C=S, C=Se, C=NR³, P(=O)(R³), SO, SO₂, NR³, O, S or CONR³ and where one or more H atoms may be replaced by D, F, Cl, Br, I, CN or NO₂, an aromatic or heteroaromatic ring system having 5 to 60 aromatic ring atoms, which may in each case be substituted by one or more radicals R³, an aryloxy or heteroaryloxy group having 5 to 60 aromatic ring atoms, which may be substituted by one or more radicals R³, or a combination of these systems, where two or more adjacent substituents R¹ may optionally form a monocyclic or polycyclic, aliphatic, aromatic or heteroaromatic ring system, which may be substituted by one or more radicals R³;
R² is selected on each occurrence, identically or differently, from the group consisting of a straight-chain alkyl group having 1 to 40 C atoms or a branched or cyclic alkyl group having 3 to 40 C atoms, each of which may be substituted by one or more radicals R³, where one or more non-adjacent CH₂ groups may be replaced by R³C=CR³, C≡C or C=O and where one or more H atoms may be replaced by D, F, Cl, Br, I, CN or NO₂, an aromatic or heteroaromatic ring system having 5 to 60 aromatic ring atoms, which may in each case be substituted by one or more radicals R³, or a combination of these systems; those R¹ and R² that are adjacent to one another in the 1,2-position may optionally form a monocyclic or polycyclic, aliphatic, aromatic or heteroaromatic ring system, which be substituted by one or more radicals R³;
R³ is selected from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN, where two or more adjacent substituents R³ may form a mono- or polycyclic, aliphatic, aromatic or heteroaromatic ring system with one another;
n is 1, 2, 3, 4, 5 or 6;
m is 1 if L³ is a single bond or a divalent group, or is 2 if L³ is a trivalent group, or is 3 if L³ is a tetravalent group;
**characterised in that** at least one group Ar¹ is present which stands for a heteroaryl group having 5 to 18 aromatic ring atoms, which may also be substituted by one or more radicals R¹, or **in that** at least one group Ar² is present which stands for an electron-deficient heteroaryl group having 5 to 18 aromatic ring atoms, where
an electron-deficient heteroaryl group is either a 5-membered heteroaryl group having at least two heteroatoms or a 6-membered heteroaryl group having at least one heteroatom, where further 6-membered aryl or 6-membered heteroaryl groups may be condensed onto these groups.

2. Compound according to Claim 1, **characterised in that** Ar³ stands, identically or differently on each occurrence, for an aryl or heteroaryl group having 5 to 10 aromatic ring atoms, which may in each case be substituted by one or more radicals R¹, preferably selected from the group consisting of benzene, pyridine, pyrimidine, pyridazine, pyrazine, furan, thiophene, pyrrole, naphthalene, phenanthrene, quinoline, isoquinoline, quinoxaline, indole, benzofuran and benzothiophene, each of which may be substituted by one or more radicals R¹.

3. Compound according to Claim 1 or 2, selected from the compounds of the formulae (9) to (18), 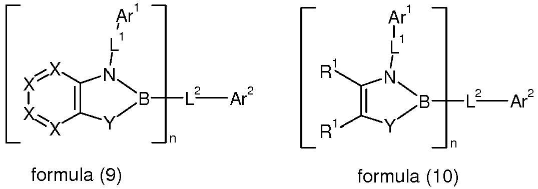 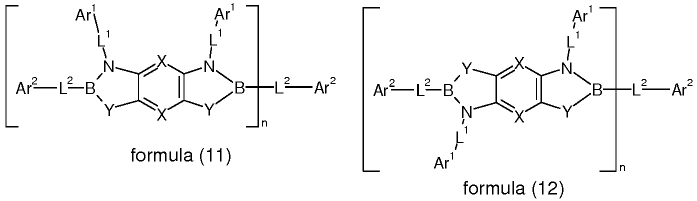 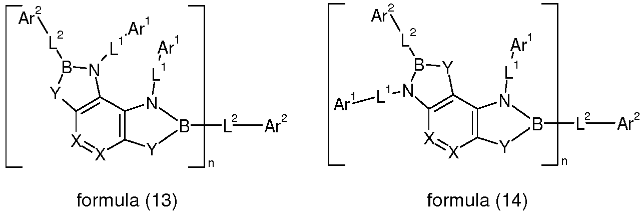 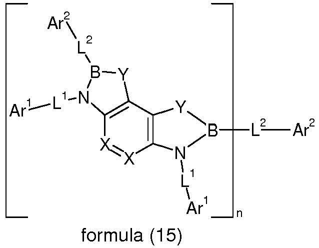 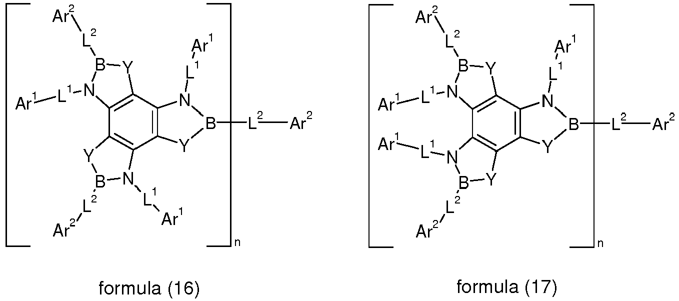 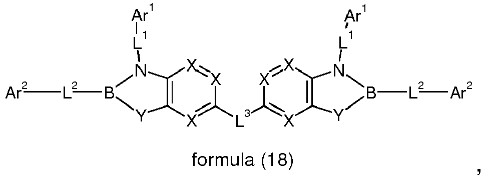 where X is on each occurrence, identically or differently, CR¹ or N and the other the symbols and indices used have the meanings given in Claim 1.

4. Compound according to one or more of Claims 1 to 3, **characterised in that** Y stands, identically or differently on each occurrence, for N-R².

5. Compound according to one or more of Claims 1 to 4, **characterised in that** L¹ or L² stands, identically or differently on each occurrence, for a single bond or a divalent arylene or heteroarylene group having 5 to 10 aromatic ring atoms, which may in each case be substituted by one or more radicals R¹, preferably for 1,2-, 1,3- or 1,4-phenylene, pyridine, pyrimidine or triazine, each of which may be substituted by one or more radicals R¹.

6. Compound according to one or more of Claims 1 to 5, **characterised in that** Ar¹ and Ar² are built up from in each case one or more groups selected from benzene, pyridine, pyrimidine, pyridazine, pyrazine, triazine, pyrrole, thiophene, furan, imidazole, indole, benzothiophene, benzofuran, benzimidazole, carbazole, dibenzofuran or dibenzothiophene, each of which may be substituted by one or more radicals R¹.

7. Compound according to one or more of Claims 1 to 6, **characterised in that** R² is selected on each occurrence, identically or differently, from the group consisting of an aromatic or heteroaromatic ring system having 5 to 30 aromatic ring atoms, which may in each case be substituted by one or more radicals R³; those R¹ and R² that are adjacent to one another in the 1,2-position may optionally form a monocyclic or polycyclic, aliphatic, aromatic or heteroaromatic ring system, which may be substituted by one or more radicals R³, preferably phenyl, naphthyl, biphenyl, terphenyl or quaterphenyl, each of which may be substituted by one or more radicals R³.

8. Compound according to one or more of Claims 3 to 7, **characterised in that** R³ is as defined in Claim 1 and furthermore:
Y is, identically or differently on each occurrence, N-R²;
L¹, L² are, identically or differently on each occurrence, a single bond or a divalent arylene or heteroarylene group having 5 to 10 aromatic ring atoms, which may in each case be substituted by one or more radicals R¹;
L³ is a single bond, O, S, NR², an alkylene group having 1 to 10 C atoms, which may be substituted by one or more radicals R³, or an aromatic or heteroaromatic ring system having 5 to 30 aromatic ring atoms, which may be substituted by one or more radicals R³;
X is on each occurrence, identically or differently, CR¹ or N, where a maximum of two symbols X in each ring stand for N and the other symbols X stand, identically or differently on each occurrence, for CR¹;
Ar¹, Ar² stand, identically or differently on each occurrence, for an aromatic or heteroaromatic ring system having 5 to 24 aromatic ring atoms, particularly preferably having 5 to 18 aromatic ring atoms, where the aromatic or heteroaromatic ring system may in each case also be substituted by one or more radicals R¹; at least one group Ar¹ is a heteroaryl group or at least one group Ar² is an electron-deficient heteroaryl group, which may be substituted by one or more radicals R¹; with the proviso that the heteroatoms are selected from N, O and/or S if Ar¹ or Ar² is a heteroaryl group;
n is 1, 2, 3 or 4, preferably 1, 2 or 3;
R¹ is selected, identically or differently on each occurrence, from the group consisting of H, D, F, CN, N(R³)₂, a straight-chain alkyl or alkoxy group having 1 to 20 C atoms or a branched or cyclic alkyl or alkoxy group having 3 to 20 C atoms or an alkenyl group having 2 to 20 C atoms, each of which may be substituted by one or more radicals R³, where one or more non-adjacent CH₂ groups may be replaced by R³C=CR³ or O and where one or more H atoms may be replaced by D or F, an aromatic or heteroaromatic ring system having 5 to 30 aromatic ring atoms, which may in each case be substituted by one or more radicals R³, an aryloxy or heteroaryloxy group having 5 to 30 aromatic ring atoms, which may be substituted by one or more radicals R³, or a combination of these systems, where two or more adjacent substituents R¹ may optionally form a monocyclic or polycyclic, aliphatic, aromatic or heteroaromatic ring system, which may be substituted by one or more radicals R³;
R² is selected on each occurrence, identically or differently, from the group consisting of an aromatic or heteroaromatic ring system having 5 to 30 aromatic ring atoms, which may in each case be substituted by one or more radicals R³; those R¹ and R² that are adjacent to one another in the 1,2-position may optionally form a monocyclic or polycyclic, aliphatic, aromatic or heteroaromatic ring system, which may be substituted by one or more radicals R³.

9. Mixture comprising at least one compound according to one or more of Claims 1 to 8 and at least one further compound.

10. Formulation comprising at least one compound or mixture according to one or more of Claims 1 to 9 and one or more solvents.

11. Process for the preparation of a compound according to one or more of Claims 1 to 8 by reaction of an aromatic ortho-diamino compound with an aryl- or heteroarylboron compound in which the boron atom is substituted by two reactive leaving groups.

12. Use of a compound or mixture according to one or more of Claims 1 to 9 in an electronic device.

13. Electronic device comprising at least one compound or mixture according to one or more of Claims 1 to 9, preferably selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes and organic plasmon emitting devices.

14. Electronic device according to Claim 13, which is an organic electroluminescent device, **characterised in that** the compound according to one or more of Claims 1 to 8 is employed as matrix material for fluorescent or phosphorescent emitters and/or in an electron-transport layer and/or in a hole-blocking layer and/or in an electron-blocking or exciton-blocking layer and/or in a hole-transport layer.

## Revendications

1. Composé de la formule (1) : 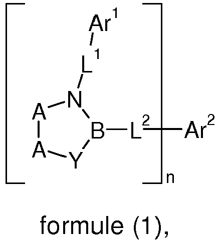 formule dans laquelle ce qui suit s'applique aux symboles et indices qui sont utilisés :
A-A est, de manière identique ou différente pour chaque occurrence, une unité de la formule (2), (3), (4), (5), (6), (7) ou (8) : 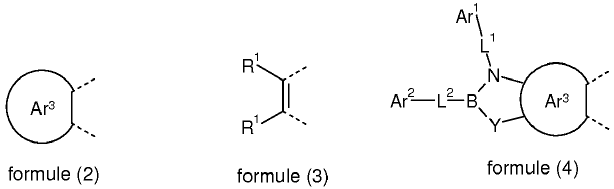 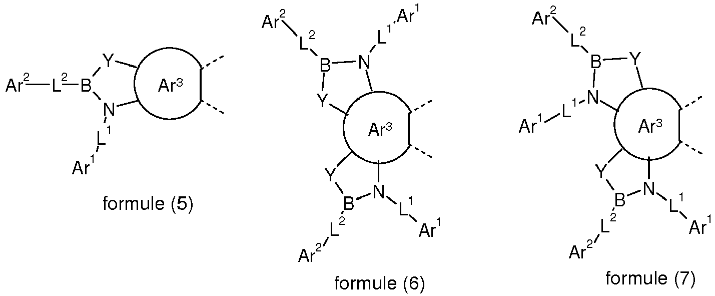 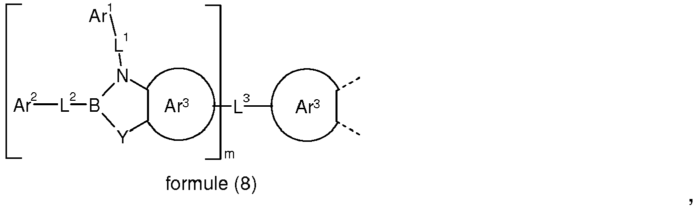 formules dans lesquelles la liaison en pointillés représente dans chaque cas le lien sur N ou sur Y ;
Y est, de manière identique ou différente pour chaque occurrence, N-R², O ou S ;
Ar¹, Ar² sont pour chaque occurrence, de manière identique ou différente, un groupe aryle ou hétéroaryle qui comporte de 5 à 18 atomes de cycle aromatique, lequel groupe peut être substitué par un radical ou par plusieurs radicaux R¹ ; étant entendu que les hétéroatomes sont sélectionnés parmi N, O et/ou S si Ar¹ ou Ar² est un groupe hétéroaryle ;
Ar³ est pour chaque occurrence, de manière identique ou différente, un groupe aryle ou hétéroaryle qui comporte de 5 à 14 atomes de cycle aromatique, lequel groupe peut être substitué par un radical ou par plusieurs radicaux R¹ ;
L¹, L² sont pour chaque occurrence, de manière identique ou différente, une liaison simple ou un système de cycle aromatique ou hétéroaromatique divalent qui comporte de 5 à 24 atomes de cycle aromatique, lequel système de cycle peut être substitué par un radical ou par plusieurs radicaux R¹ ;
L³ est une liaison simple ou un groupe divalent, trivalent ou tétravalent ;
R¹ est sélectionné pour chaque occurrence, de manière identique ou différente, parmi le groupe qui est constitué par H, D, F, Cl, Br, I, CN, NO₂, N(R³)₂, C(=O)R³, un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, groupes dont chacun peut être substitué par un radical ou par plusieurs radicaux R³, où un ou plusieurs groupe(s) CH₂ non adjacents peut/peuvent être remplacé(s) par R³C=CR³, C≡C, Si(R³)₂, Ge(R³)2, Sn(R³)2, C=O, C=S, C=Se, C=NR³, P(=O)(R³), SO, SO₂, NR³, O, S ou CONR³ et où un ou plusieurs atome(s) de H peut/peuvent être remplacé(s) par D, F, Cl, Br, I, CN ou NO₂, un système de cycle aromatique ou hétéroaromatique qui comporte de 5 à 60 atomes de cycle aromatique, lequel système de cycle peut dans chaque cas être substitué par un radical ou par plusieurs radicaux R³, un groupe aryloxy ou hétéroaryloxy qui comporte de 5 à 60 atomes de cycle aromatique, lequel groupe peut être substitué par un radical ou par plusieurs radicaux R³, ou une combinaison de ces systèmes, où deux substituants R¹ adjacents ou plus peuvent en option former un système de cycle aliphatique, aromatique ou hétéroaromatique monocyclique ou polycyclique, lequel système de cycle peut être substitué par un radical ou par plusieurs radicaux R³ ;
R² est sélectionné pour chaque occurrence, de manière identique ou différente, parmi le groupe qui est constitué par un groupe alkyle en chaîne droite qui comporte de 1 à 40 atome(s) de C ou un groupe alkyle ramifié ou cyclique qui comporte de 3 à 40 atomes de C, groupes dont chacun peut être substitué par un radical ou par plusieurs radicaux R³, où un ou plusieurs groupe(s) CH₂ non adjacents peut/peuvent être remplacé(s) par R³C=CR³, C≡C ou C=O et où un ou plusieurs atome(s) de H peut/peuvent être remplacé(s) par D, F, Cl, Br, I, CN ou NO₂, un système de cycle aromatique ou hétéroaromatique qui comporte de 5 à 60 atomes de cycle aromatique, lequel système de cycle peut dans chaque cas être substitué par un radical ou par plusieurs radicaux R³, ou une combinaison de ces systèmes ; les R¹ et R² qui sont adjacents l'un à l'autre ou les uns aux autres au niveau des positions 1, 2 peuvent en option former un système de cycle aliphatique, aromatique ou hétéroaromatique monocyclique ou polycyclique, lequel système de cycle peut être substitué par un radical ou par plusieurs radicaux R³ ;
R³ est sélectionné parmi le groupe qui est constitué par H, D, F, CN, un radical hydrocarbone aliphatique qui comporte de 1 à 20 atome(s) de C, un système de cycle aromatique ou hétéroaromatique qui comporte de 5 à 30 atomes de cycle aromatique, système de cycle dans lequel un ou plusieurs atome(s) de H peut/ peuvent être remplacé(s) par D, F, Cl, Br, I ou CN, où deux substituants R³ adjacents ou plus peuvent former un système de cycle aliphatique, aromatique ou hétéroaromatique monocyclique ou polycyclique l'un avec l'autre ou les uns avec les autres ;
n est 1, 2, 3, 4, 5 ou 6 ;
m est 1 si L³ est une liaison simple ou un groupe divalent, ou est 2 si L³ est un groupe trivalent, ou est 3 si L³ est un groupe tétravalent ;
**caractérisé en ce qu'**au moins un groupe Ar¹ est présent, lequel représente un groupe hétéroaryle qui comporte de 5 à 18 atomes de cycle aromatique, lequel groupe peut également être substitué par un radical ou par plusieurs radicaux R¹, ou **en ce qu'**au moins un groupe Ar² est présent, lequel représente un groupe hétéroaryle déficient en électrons qui comporte de 5 à 18 atomes de cycle aromatique, où un groupe hétéroaryle déficient en électrons est soit un groupe hétéroaryle à 5 éléments qui comporte au moins deux hétéroatomes, soit un groupe hétéroaryle à 6 éléments qui comporte au moins un hétéroatome, où en outre des groupes aryle à 6 éléments ou hétéroaryle à 6 éléments peuvent être condensés sur ces groupes.

2. Composé selon la revendication 1, **caractérisé en ce que** Ar³ représente, de manière identique ou différente pour chaque occurrence, un groupe aryle ou hétéroaryle qui comporte de 5 à 10 atomes de cycle aromatique, lequel groupe peut dans chaque cas être substitué par un radical ou par plusieurs radicaux R¹, de préférence sélectionné(s) parmi le groupe qui est constitué par benzène, pyridine, pyrimidine, pyridazine, pyrazine, furane, thiophène, pyrrole, naphtalène, phénanthrène, quinoline, isoquinoline, quinoxaline, indole, benzofurane et benzothiophène, dont chacun peut être substitué par un radical ou par plusieurs radicaux R¹.

3. Composé selon la revendication 1 ou 2, sélectionné parmi les composés des formules (9) à (18) : 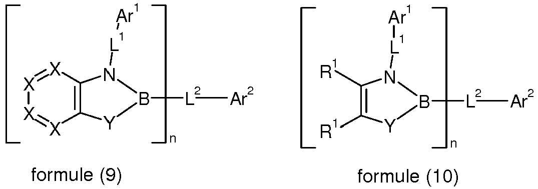 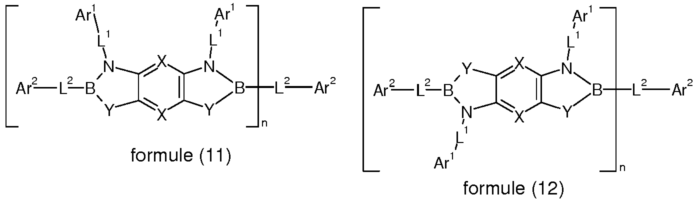 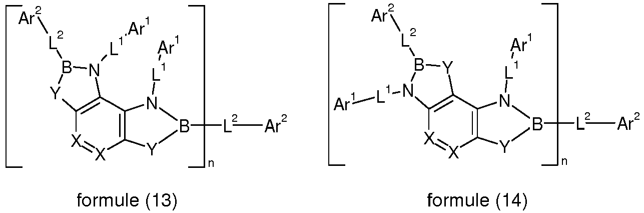 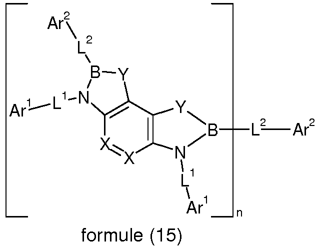 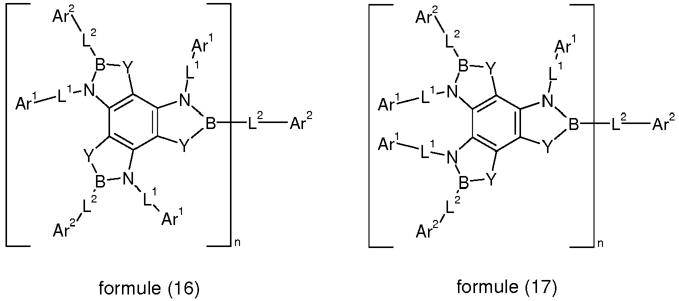 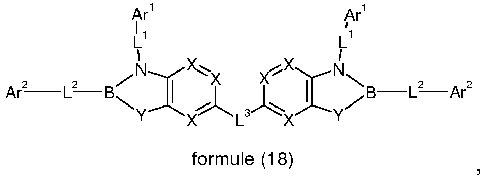 formules dans lesquelles X est pour chaque occurrence, de manière identique ou différente, CR¹ ou N et les autres symboles et indices qui sont utilisés présentent les significations qui ont été données selon la revendication 1.

4. Composé selon une ou plusieurs des revendications 1 à 3, **caractérisé en ce que** Y représente, de manière identique ou différente pour chaque occurrence, N-R².

5. Composé selon une ou plusieurs des revendications 1 à 4, **caractérisé en ce que** L¹ ou L² représente, de manière identique ou différente pour chaque occurrence, une liaison simple ou un groupe arylène ou hétéroarylène divalent qui comporte de 5 à 10 atomes de cycle aromatique, lequel groupe peut dans chaque cas être substitué par un radical ou par plusieurs radicaux R¹, de préférence 1,2-, 1,3- ou 1,4-phénylène, pyridine, pyrimidine ou triazine, dont chacun peut être substitué par un radical ou par plusieurs radicaux R¹.

6. Composé selon une ou plusieurs des revendications 1 à 5, **caractérisé en ce que** Ar¹ et Ar² sont constitués dans chaque cas à partir d'un ou de plusieurs groupe(s) qui est/sont sélectionné(s) parmi benzène, pyridine, pyrimidine, pyridazine, pyrazine, triazine, pyrrole, thiophène, furane, imidazole, indole, benzothiophène, benzofurane, benzimidazole, carbazole, dibenzofurane ou dibenzothiophène, dont chacun peut être substitué par un radical ou par plusieurs radicaux R¹.

7. Composé selon une ou plusieurs des revendications 1 à 6, **caractérisé en ce que** R² est sélectionné pour chaque occurrence, de manière identique ou différente, parmi le groupe qui est constitué par un système de cycle aromatique ou hétéroaromatique qui comporte de 5 à 30 atomes de cycle aromatique, lequel système de cycle peut dans chaque cas être substitué par un radical ou par plusieurs radicaux R³ ; les R¹ et R² qui sont adjacents l'un à l'autre ou les uns aux autres au niveau des positions 1, 2 peuvent en option former un système de cycle aliphatique, aromatique ou hétéroaromatique monocyclique ou polycyclique, lequel système de cycle peut être substitué par un radical ou par plusieurs radicaux R³, de préférence phényle, naphtyle, biphényle, terphényle ou quaterphényle, radicaux dont chacun peut être substitué par un radical ou par plusieurs radicaux R³.

8. Composé selon une ou plusieurs des revendications 1 à 7, **caractérisé en ce que** R³ est comme il a été défini selon la revendication 1 et en outre :
Y est, de manière identique ou différente pour chaque occurrence, N-R² ;
L¹, L² sont, de manière identique ou différente pour chaque occurrence, une liaison simple ou un groupe arylène ou hétéroarylène divalent qui comporte de 5 à 10 atomes de cycle aromatique, lequel groupe peut dans chaque cas être substitué par un radical ou par plusieurs radicaux R¹ ;
L³ est une liaison simple, O, S, NR², un groupe alkylène qui comporte de 1 à 10 atome(s) de C, lequel groupe peut être substitué par un radical ou par plusieurs radicaux R³, ou un système de cycle aromatique ou hétéroaromatique qui comporte de 5 à 30 atomes de cycle aromatique, lequel système de cycle peut être substitué par un radical ou par plusieurs radicaux R³ ;
X est pour chaque occurrence, de manière identique ou différente, CR¹ ou N, où un maximum de deux symboles X dans chaque cycle représentent N et les autres symboles X représentent, de manière identique ou différente pour chaque occurrence, CR¹ ;
Ar¹, Ar² représentent, de manière identique ou différente pour chaque occurrence, un système de cycle aromatique ou hétéroaromatique qui comporte de 5 à 24 atomes de cycle aromatique, de façon particulièrement préférable qui comporte de 5 à 18 atomes de cycle aromatique, où le système de cycle aromatique ou hétéroaromatique peut dans chaque cas également être substitué par un radical ou par plusieurs radicaux R¹ ; au moins un groupe Ar¹ est un groupe hétéroaryle ou au moins un groupe Ar² est un groupe hétéroaryle déficient en électrons, lequel groupe peut être substitué par un radical ou par plusieurs radicaux R¹ ; étant entendu que les hétéroatomes sont sélectionnés parmi N, O et/ou S si Ar¹ ou Ar² est un groupe hétéroaryle ;
n est 1, 2, 3 ou 4, de préférence 1, 2 ou 3 ;
R¹ est sélectionné, de manière identique ou différente pour chaque occurrence, parmi le groupe qui est constitué par H, D, F, CN, N(R³)₂, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle qui comporte de 2 à 20 atomes de C, groupes dont chacun peut être substitué par un radical ou par plusieurs radicaux R³, où un ou plusieurs groupe(s) CH₂ non adjacents peut/peuvent être remplacé(s) par R³C=CR³ ou O et où un ou plusieurs atome(s) de H peut/peuvent être remplacé(s) par D ou F, un système de cycle aromatique ou hétéroaromatique qui comporte de 5 à 30 atomes de cycle aromatique, lequel système de cycle peut dans chaque cas être substitué par un radical ou par plusieurs radicaux R³, un groupe aryloxy ou hétéroaryloxy qui comporte de 5 à 30 atomes de cycle aromatique, lequel groupe peut être substitué par un radical ou par plusieurs radicaux R³, ou une combinaison de ces systèmes, où deux substituants R¹ adjacents ou plus peuvent en option former un système de cycle aliphatique, aromatique ou hétéroaromatique monocyclique ou polycyclique, lequel système de cycle peut être substitué par un radical ou par plusieurs radicaux R³ ;
R² est sélectionné pour chaque occurrence, de manière identique ou différente, parmi le groupe qui est constitué par un système de cycle aromatique ou hétéroaromatique qui comporte de 5 à 30 atomes de cycle aromatique, lequel système de cycle peut dans chaque cas être substitué par un radical ou par plusieurs radicaux R³ ; les R¹ et R² qui sont adjacents l'un à l'autre ou les uns aux autres au niveau des positions 1, 2 peuvent en option former un système de cycle aliphatique, aromatique ou hétéroaromatique monocyclique ou polycyclique, lequel système de cycle peut être substitué par un radical ou par plusieurs radicaux R³.

9. Mélange comprenant au moins un composé selon une ou plusieurs des revendications 1 à 8 et au moins un autre composé.

10. Formulation comprenant au moins un composé ou une mélange selon une ou plusieurs des revendications 1 à 9 et un ou plusieurs solvant(s).

11. Procédé pour la préparation d'un composé selon une ou plusieurs des revendications 1 à 8 par réaction d'un composé ortho-diamino aromatique avec un composé arylbore ou hétéroarylbore dans lequel l'atome de bore est substitué par deux groupes partants réactifs.

12. Utilisation d'un composé ou d'une mélange selon une ou plusieurs des revendications 1 à 9 dans un dispositif électronique.

13. Dispositif électronique comprenant au moins un composé ou une mélange selon une ou plusieurs des revendications 1 à 9, de préférence sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires sensibilisées par colorant(s) organiques, les détecteurs optiques organiques, les photorécepteurs organiques, les dispositifs à extinction de champ organiques, les cellules électrochimiques à émission de lumière, les diodes laser organiques et les dispositifs à émission de plasmons organiques.

14. Dispositif électronique selon la revendication 13, lequel est un dispositif électroluminescent organique, **caractérisé en ce que** le composé selon une ou plusieurs des revendications 1 à 8 est utilisé en tant que matériau de matrice pour des émetteurs fluorescents ou phosphorescents et/ou dans une couche de transport d'électrons et/ou dans une couche de blocage de trous et/ou dans une couche de blocage d'électrons ou une couche de blocage d'excitons et/ou dans une couche de transport de trous.
